(19) Europäisches Patentamt
European Patent Office
Office européen des brevets

(11) EP 0 668 904 B1

(12) **FASCICULE DE BREVET EUROPEEN**

(45) Date de publication et mention de la délivrance du brevet:
**07.11.2001 Bulletin 2001/45**

(21) Numéro de dépôt: **94921073.6**

(22) Date de dépôt: **28.07.1994**

(51) Int Cl.⁷: **C11D 3/50**, C11D 3/386, C07C 69/007

(86) Numéro de dépôt international:
**PCT/IB94/00228**

(87) Numéro de publication internationale:
**WO 95/04809 (16.02.1995 Gazette 1995/08)**

(54) **PROCEDE DE PARFUMAGE DE TEXTILES**

PARFÜMIERUNGSVERFAHREN VON GEWEBEN

FABRIC SCENTING METHOD

(84) Etats contractants désignés:
**CH DE FR GB LI NL**

(30) Priorité: **09.08.1993 CH 236393**

(43) Date de publication de la demande:
**30.08.1995 Bulletin 1995/35**

(73) Titulaire: **FIRMENICH SA**
**1211 Genève 8 (CH)**

(72) Inventeurs:
- **PAGET, Walter**
  **CH-1290 Versoix (CH)**
- **REICHLIN, Daniel**
  **CH-1232 Confignon (CH)**
- **SNOWDEN, Roger, L.**
  **F-74580 Viry (FR)**
- **WALBORSKY, Eric, C.**
  **Bridgewater, NJ 08807 (US)**
- **VIAL, Christian**
  **CH-1219 Le Lignon (CH)**

(74) Mandataire:
**Salvaterra-Garcia, Maria de Lurdes et al**
**Firmenich SA**
**Département des Brevets**
**Case Postale 239**
**1211 Genève 8 (CH)**

(56) Documents cités:
**EP-A- 0 397 245** **EP-A- 0 430 315**
**EP-A- 0 513 470**

- **Beilsteins Handbuch der organischen Chemie, IIIEW, Band 6, pp1708-10, Syst.Nr. 529.**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 668 904 B1

## Description

### Domaine technique et technique antérieure

La présente invention a trait à la parfumerie et, plus particulièrement, à des détergents et adoucissants textiles parfumés améliorés.

On connaît, depuis un certain nombre d'années, l'utilisation d'enzymes dans des détergents pour le linge, dans le but d'en améliorer l'efficacité. Parmi ces enzymes, les lipases sont particulièrement préférées, en raison de leur capacité d'hydrolyser les matières grasses sur du linge sale et de faciliter ainsi leur nettoyage. Cependant, il est connu que des problèmes de mauvaises odeurs peuvent parfois surgir dans certaines conditions d'application. Pour palier ces effets malodorants, une méthode a été proposée (voir par exemple EP 430 315) consistant à choisir soigneusement les ingrédients parfumants incorporés dans les détergents et qui, par suite du lavage, sont déposés sur les tissus. Un parfumage approprié de ces détergents apparaît donc comme extrêmement important.

D'autre part, il serait souhaitable que ces détergents et adoucissants textiles parfumés puissent impartir aux textiles une odeur de longue durée, de sorte que l'utilisateur ait une perception de cette odeur même longtemps après le lavage des textiles et le séchage subséquent. A cet effet, il est connu d'utiliser dans les détergents et adoucissants textiles des ingrédients parfumants ayant une bonne tenacité sur les tissus, c'est-à-dire des ingrédients dont l'odeur, une fois impartie au textile lors du lavage, puisse être perçue par la suite par le consommateur pendant plusieurs jours. Toutefois, de nombreuses substances parfumantes sont connues pour posséder des odeurs extrêmement agréables, et notamment une qualité dite de "fraîcheur" souvent associée à la notion de propreté, lesquelles substances sont malheureusement peu tenaces, ou même pas tenaces du tout, sur du linge, de sorte que leur effet parfumant n'est perçu que brièvement, au plus pendant quelques heures après les opérations de lavage et de séchage. Or, il serait bien entendu très souhaitable que l'on puisse prolonger l'effet odorant de telles substances et ainsi la "fraîcheur" du linge pendant plusieurs jours.

La présente invention apporte précisément une solution nouvelle à ce problème. Nous avons maintenant découvert, de façon inattendue, un meilleur procédé de parfumage de textiles lavés avec des détergents contenant des lipases. Nous avons en effet pu établir qu'en ajoutant des ingrédients particuliers au détergent pour le linge, et/ou à l'adoucissant textile appliqué par la suite, on pouvait clairement améliorer l'odeur du linge traité avec ces produits et prolonger, de façon remarquable, la fragrance de celui-ci après séchage.

### Exposé de l'invention

Un premier objet de l'invention est donc de fournir un procédé pour le parfumage de textiles soumis au lavage en présence d'un détergent contenant une lipase, suivi facultativement d'un traitement avec un adoucissant textile, le procédé étant caractérisé en ce que ledit détergent et/ou ledit adoucissant contiennent un composé de formule

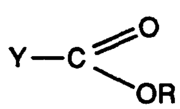

(I)

dans laquelle

a. R représente un radical dérivé d'un alcool odorant de formule ROH et Y représente un radical alkyle de $C_7$ à $C_{24}$, linéaire ou ramifié, saturé ou insaturé, ou un groupe $-(CH_2)_nCOOR$ dans lequel R est défini comme ci-dessus et n est un nombre entier de 0 à 6 ; ou

b. Y représente un radical alkyle de $C_7$ à $C_{24}$, linéaire ou ramifié, saturé ou insaturé, et R représente un groupe de formule

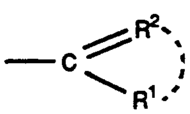

dans laquelle, soit $R^1$ représente l'hydrogène et $R^2$ représente un radical alkylidène dérivé d'un aldéhyde odorant de formule

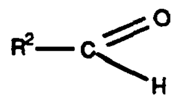

soit $R^2$ représente un radical alkylidène et $R^1$ un radical alkyle, $R^1$ et $R^2$ étant dérivés d'une cétone odorante de formule

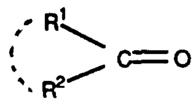

et pouvant faire partie d'un cycle tel qu'indiqué par la ligne pointillée, contenant de 5 à 18 atomes de carbone, éventuellement substitué.

Par un radical alkylidène dérivé d'une cétone ou aldéhyde odorant, on entend ici un radical qui, lors de la transformation de l'énol-ester (I) en ledit aldéhyde ou cétone, régénère le groupe R correspondant substituant de cet aldéhyde ou cétone. Ainsi, par exemple, lorsque l'aldéhyde odorant est le 3,7-diméthyl-6-octénal (R est 3,7-diméthyl-6-octényle), le radical alkylidène correspondant dans l'énol-ester (I) est le 3,7-diméthyl-1,6-octadiényle.

Selon une variante de l'invention, on fournit un procédé pour le parfumage de textiles soumis au lavage en présence d'un détergent contenant une lipase, lequel procédé comprend le traitement desdits textiles, après le cycle de lavage, avec un adoucissant textile contenant un composé de formule (I) telle que définie auparavant.

Par "alcool odorant", "aldéhyde odorant" et "cétone odorante" on entend ici un quelconque alcool, aldéhyde, respectivement cétone d'usage courant en parfumerie, lequel est capable d'impartir, lors du processus de lavage et/ou lors du traitement avec un adoucissant, une odeur à des textiles. Nous avons en effet découvert que l'effet parfumant de tels composés pouvait être remarquablement amélioré, et notamment leur diffusion prolongée, si on les remplaçait dans le détergent ou adoucissant textile employé par le composé (I) correspondant.

Ce résultat est d'autant plus surprenant que les composés (I) sont, soit eux-mêmes dépourvus d'odeur, soit ils possèdent des odeurs faibles et sans caractère, donc sans intérêt apparent pour la parfumerie. Or, lorsqu'utilisés selon l'invention, ils sont non seulement capables d'impartir aux textiles l'odeur caractéristique de l'alcool, aldéhyde ou cétone correspondants, mais également d'en prolonger l'effet de diffusion, de façon que cette odeur se développe pendant des périodes de temps beaucoup plus longues que dans le cas où l'on ajoute lesdits alcool, aldéhyde ou cétone correspondants directement aux détergents ou adoucissants textiles. Ainsi, l'emploi des composés (I) selon le procédé de l'invention se traduit par une augmentation de la substantivité des alcools, aldéhydes ou cétones correspondants.

Les avantages du procédé apparaissent encore plus évidents dans le cas des nombreux alcools odorants que l'on sait très peu tenaces sur des textiles soumis à lavage et dont l'odeur, bien que distinctement perçue à la sortie de la machine à laver, ne demeure pas sur le linge et ne peut plus être perçue au-delà de 12 à 24 h.

De nombreux exemples de tels alcools peuvent être trouvés dans l'art antérieur, dont quelques uns ne contribuent à l'odeur des tissus que pendant très peu de temps, et sans doute beaucoup d'autres seront-ils découverts dans le futur. Pour tous ces alcools, le procédé selon l'invention permet d'améliorer de façon surprenante leur performance odorante sur les textiles, en prolongeant le temps de diffusion de leurs notes caractéristiques et ainsi le temps pendant lequel ils contribuent significativement à l'odeur globale. Nous avons en effet constaté que l'utilisation des composés (I) correspondants dans les détergents et/ou adoucissants textiles permettait d'obtenir un effet parfumant équivalent à celui que l'on aurait observé avec la diffusion prolongée ("slow-release") de l'alcool, si une telle diffusion avait été possible, ce qui n'est pas le cas dans la pratique.

Bien entendu, on ne peut pas donner une liste exhaustive des alcools de formule ROH connus à ce jour qui sont capables d'impartir des odeurs agréables aux textiles traités avec des produits de lessive parfumés à l'aide desdits alcools, et dont l'effet parfumant peut être remarquablement amélioré selon l'invention. Cependant, à titre d'exemple, on peut citer des alcools tels que l'alcool anisique, l'alcool cinnamique, l'alcool fenchylique, le dec-9-én-1-ol, le phénéthylol, le citronellol, le 3-méthyl-5-phényl-1-pentanol (origine : Firmenich SA, Genève, Suisse), le Mayol ® (7-p-menthan-1-ol; origine : Firmenich SA, Genève, Suisse), le dihydromyrcenol (2,6-diméthyl-oct-7-én-2-ol), le géraniol (3,7-diméthyl-octa-2,6-dién-1-ol), le (Z)-hex-3-én-1-ol, le 1-hexanol, le 2-hexanol, le 5-éthyl-2-nonanol, le nona-2,6-dién-1-ol, le bornéol, le oct-1-én-3-ol, le 4-cyclohexyl-2-méthyl-2-butanol (origine: Firmenich SA, Genève, Suisse), le 2-méthyl-4-phényl-2-butanol, le 2-méthyl-1-phényl-2-propanol, le cyclométhylcitronellol, le décanol, le dihydroeugénol, le 8-p-menthanol, le 3,7-diméthyl-1-octanol, le 2,6-diméthyl-2-heptanol, le dodécanol, l'eucalyptol, l'eugénol, le Florol® (tétrahydro-2-isobutyl-4-méthyl-4(2H)-pyranol ; origine : Firmenich SA, Genève, Suisse), l'isoeugénol, le linalol, le Tarragol ® (2-méthoxy-4-propyl-1-cylohexanol ; origine : Firmenich SA, Genève, Suisse), le terpinéol, le tétrahydromuguol, le 3,7-diméthyl-3-octanol et le Lyral ® (3 et 4-(4-hydroxy-4-méthylpentyl)-cyclohex-3-ène-1-carbaldéhyde ; origine : International Flavors and Fragrances, USA).

Il est bien évident, cependant, que le procédé de l'invention est tout à fait général et peut avoir trait à bien d'autres alcools que l'homme du métier est à même de choisir de par ses connaissances de l'art et en fonction de l'effet olfactif qu'il désire obtenir.

Des considérations analogues s'appliquent aux aldéhydes de formule

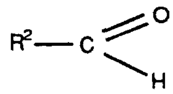

et aux cétones de formule

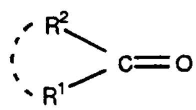

Le procédé de l'invention se révèle tout à fait avantageux dès lors que des composés tels que susmentionnés présentent une faible ténacité sur tissu, et nombreux sont les aldéhydes et cétones odorants dont la performance sur les textiles se trouve nettement améliorée lorsque ces composés exercent leur activité parfumante par le biais des énolesters de formule (I) correspondants.

De nouveau, bien que l'on ne puisse pas citer de façon exhaustive tous les aldéhydes et cétones odorants pouvant être employés selon le procédé de l'invention, on peut mentionner à titre d'exemple des composés tels que les aldéhydes $C_6$ à $C_{12}$, l'aldéhyde hydratropique, le méthyl nonyl acétaldéhyde, l'aldéhyde phénylpropionique, l'Acropal ® [3- ou 4-(4-méthyl-3-pentényl)-3-cyclohexène-1-carbaldéhyde ; origine : Givaudan-Roure, Vernier, Suisse], le 2-méthyldécanal, le 4-isopropyl-1-benzèneacétaldéhyde, le (4-méthyl-1-phényl)acétaldéhyde, le Z-6-nonénal, le citral, le citronellal, le 9-décénal, le 3-(4-isopropyl-1-phényl)-2-méthylpropanal (origine: Firmenich SA, Genève, Suisse), le (E,E)-2,4-heptadiénal, le (E,E)-2,4-nonadiénal, le (E,E)-2,4-décadiénal, le 5,9-diméthyl-4,9-décadiénal, le (Z)-6-octénal, le Farenal ® (2,6,10-triméthyl-9-undécénal ; origine : Givaudan-Roure, Vernier, Suisse), le Foliaver ® [3-(4-méthoxy-1-phényl)-2-méthylpropanal ; origine : International Flavors and Fragrances, USA], l'Heliopropanal ® [3-(1,3-benzodioxol-5-yl)-2-méthylpropanal ; origine : International Flavors and Fragrances, USA], le (Z)-4-hepténal, le 3,5,5-triméthylhexanal (origine : International Flavors and Fragrances, USA), le (4-méthyl-1-phénoxy)acétaldéhyde, l'hydroxycitronellal, l'isocyclocitral (origine : International Flavors and Fragrances, USA), le Lilial ® [3-(4-tert-butyl-1-phényl)-2-méthylpropanal ; origine : Givaudan-Roure, Vernier, Suisse], le 1-p-menthène-9-carbaldéhyde (origine : Firmenich SA, Genève, Suisse), le Lyral ® [3- et 4-(4-hydroxy-4-méthylpentyl)-3-cydohexène-1-carbaldéhyde ; origine : International Flavors and Fragrances, USA], le 2,6-diméthyl-5-hepténal, le 1-p-menthèn-9-al, le (E)-2-octénal, le (2E,6Z)-2,6-nonadiénal, le 3-méthyl-5-phénylpentanal, le (E)-4-décénal, le (E)-2-undécénal, le 3,7-diméthyloctanal, le Zestover (2,4-diméthyl-3-cyclohexène-1-carbaldéhyde ; origine : Firmenich SA, Genève, Suisse), le 3-phénylbutanal, le Scentenal ® (octahydro-5-méthoxy-4,7-méthano-lH-indène-2-carboxaldéhyde ; origine : Firmenich SA, Genève, Suisse), le 2,5,9-triméthyl-4,9-décadiénal, l'aldéhyde Intreleven (undécénal; origine: International Flavors and Fragrances, USA), l'aldéhyde 4-méthyl-phényl-propionique, la 4-(4-hydroxy-1-phényl)-2-butanone, la benzylacétone, les ionones, le 3-(4-tert-butyl-1-phényl)propanal, la carvone, le 3,7-diméthyl-1,1-bis(11-méthyldodécyloxy)-2,6-octadiène, la muscone, la 2-pentyl-1-cydopentanone, l'éthyl amyl cétone, l'éthyl pentyl cétone, la 2-heptyl-1-cyclopentanone, la géranylacétone, l'Iralia ® (méthylionone; origine: Firmenich SA, Genève, Suisse), l'Iso E super [1-(octahydro-2,3,8,8-tétraméthyl-2-naphtalényl)-1-éthanone ; origine : International Flavors and Fragrances, USA], la 6-méthyl-5-heptén-2-one, le jasmonate de méthyle, la méthyl hexyl cétone, la méthyl pentyl cétone, la méthylnonyl cétone, la cis-jasmone, l'Hedione ® (dihydrojasmonate de méthyle ; origine : Firmenich SA, Genève, Suisse), la civettone, la 4-(1,1-diméthylpropyl)-1-cyclohexanone, l'Exaltone ® (cyclopentadécanone ; origine : Firmenich SA, Genève, Suisse), le 2,6,6-triméthyl-2-cyclohexène-1,4-dione, la p-tert-butylcyclohexanone, la tricyclo[6.2.1.0$^{2,7}$]undéc-9-én-3-one (origine : Firmenich SA, Genève, Suisse), la 10,10-diméthyl-tricydo [7.1.1.0$^{2,7}$]undéc-2-én-4-one (origine : Firmenich SA, Genève, Suisse), le Vertofix coeur (origine: International Flavors and Fragrances, USA), la perhydro-5,5,8a-triméthyl-2-naphtalénone (origine: Firmenich SA, Genève, Suisse) ou la 5-méthyl-exo-tricyclo[6.2.1.0$^{2,7}$]undécan-4-one.

Il convient de noter, par ailleurs, que le procédé de l'invention est d'un usage bien plus général que le parfumage amélioré des textiles traités avec des produits de lessive contenant des lipases. Il constitue, en effet, un procédé général de lavage desdits textiles, qui se révèle également avantageux dès lors que l'on désire améliorer l'activité de certains agents, odorants ou autres, couramment présents dans les produits de lessive, à savoir détergents et adoucissants textiles. On sait, par exemple, que ces produits sont des milieux très agressifs, dans lesquels bon nombre d'ingrédients parfumants, et notamment d'aldéhydes odorants, se révèlent instables et ne peuvent donc être utilisés pour le parfumage desdits produits et des textiles lavés avec ces produits. L'utilisation selon l'invention des esters et énol-esters de formule (I) correspondants peut palier à ce problème dès lors que ces énol-esters se révèlent plus stables. Il est clair également que l'on peut profiter du procédé de l'invention pour améliorer et prolonger l'action d'agents bactéricides, notamment des alcools, odorants ou pas.

Ainsi, le procédé selon l'invention est en effet un procédé général de traitement de textiles lors du lavage de ces derniers avec un détergent contenant une lipase, lequel lavage peut être suivi d'un traitement avec un adoucissant textile, selon lequel procédé tout alcool, aldéhyde ou cétone couramment utilisé dans des détergents ou adoucissants textiles pour son activité odorante, bactéricide ou autre, peut être remplacé par le composé (I) correspondant, dans le but d'en améliorer l'activité.

Les composés (I) (à l'exception du 2-phényléthyle, de l'adipate de n-hexyle, de l'adipate de cyclohexyle, de l'adipate de n-hexylcyclohexyle) sont des entités chimiques nouvelles et font ainsi également l'objet de l'invention. Parmi ces composés, ceux dans lesquels Y représente un radical alkyle de $C_7$ à $C_{24}$, linéaire ou ramifié, saturé ou insaturé, sont des esters ou énol-esters d'acides gras qui ne possèdent pas eux-mêmes une odeur intéressante, mais qui, lorsqu'utilisés selon le procédé de l'invention, sont capables d'impartir aux textiles les fragrances typiques des alcools, aldéhydes et cétones correspondants, définis plus haut. Ces composés, en particulier les dérivés ayant de 12 à 16 atomes de carbone, sont préférés selon l'invention en raison des résultats particulièrement avantageux obtenus lors de leur utilisation.

En ce qui concerne les composés (I) pour lesquels Y représente un groupe $-(CH_2)_nCOOR$, R étant défini comme ci-dessus et n étant un nombre entier de 0 à 6, il s'agit de diesters nouveaux qui, soit sont dépourvus d'odeur, soit possèdent des odeurs sans intérêt particulier en parfumerie. Cependant, ils ont montré un comportement semblable à celui des composés (I) cités ci-dessus lors de leur utilisation dans le contexte de la présente invention.

Les composés (I) de l'invention peuvent être ajoutés aux détergents et adoucissants textiles, soit en l'état, soit en mélange avec d'autres ingrédients parfumants, solvants ou adjuvants d'usage courant en parfumerie. Les concentrations dans lesquelles ils peuvent être ajoutés aux détergents et adoucissants textiles selon l'invention ont des valeurs courantes dans l'art pour ce type de produit. L'homme du métier est à même de choisir ces valeurs en fonction de la nature du produit à parfumer et de l'effet olfactif qu'il désire obtenir. A titre d'exemple, on peut citer des concentrations de l'ordre de 0,01 à 1%, voire même jusqu'à 5%, en poids de composé (I), par rapport au poids de composition détergente ou d'adoucissant textile.

Les détergents et adoucissants textiles selon l'invention peuvent se présenter sous forme de poudres ou de solides granulaires, de barres, de pâtes ou encore de liquides, aqueux ou non-aqueux et contiennent des ingrédients courants dans ce type de produit. C'est ainsi que les détergents peuvent contenir, en plus de la lipase (voir, par exemple, EP 430 315 pour une description détaillée du type de lipases pouvant être utilisées selon la présente invention) par exemple des composés actifs anioniques, cationiques, zwittérioniques ou non-ioniques, ainsi que des agents de remplissage, blanchissants, séquestrants et autres ingrédients d'usage courant dans les bases détergentes destinées au lavage du linge. Une description détaillée des compositions détergentes de base qui se prêtent à l'utilisation selon le procédé de l'invention est ici superflue. De nombreux exemples de telles compositions peuvent être trouvés dans l'art et notamment dans la littérature citée par exemple dans la demande de brevet européen mentionnée plus haut ou encore dans la demande de brevet européen EP 397 245. A titre d'exemple, une base détergente de ce type, à laquelle on ajoute la lipase dans la concentration désirée, peut avoir la composition suivante (origine : Henkel KGaA, Düsseldorf, Allemagne) :

| Ingrédients | % en poids |
|---|---|
| Sulfonate linéaire de sodium alkyl benzène (longueur moyenne de la chaîne d'alcanes : $C_{11,5}$) | 8,0 |
| Alcool de suif éthoxylé (14 EO) | 2,9 |
| Savon de sodium (longueur de chaîne $C_{12-16}$ : 13-26%<br>$C_{18-22}$ : 74-87%) | 3,5 |
| Triphosphate de sodium | 43,8 |
| Silicate de sodium ($SiO_2$ : $Na_2O$=3,3:1) | 7,5 |
| Silicate de magnésium | 1,9 |

(suite)

| Ingrédients | % en poids |
|---|---|
| Carboxyméthylcellulose | 1,2 |
| Ethylènediaminetétraacétate de sodium | 0,2 |
| Sulfate de sodium | 21,2 |
| Eau | 9,8 |
| Total | 100,0 |

Des considérations semblables s'appliquent aux bases adoucissantes textiles selon l'invention, lesquelles contiendront typiquement des ingrédients adoucissants cationiques du type de ceux cités dans EP 397 245 ou dans la littérature mentionnée dans cette référence.

Les composés de l'invention peuvent être préparés par des méthodes de synthèse conventionnelles. Par exemple, les dérivés d'alcools odorants ont été préparés par la méthode d'estérification résumée dans le schéma de réaction suivant :

Schéma I

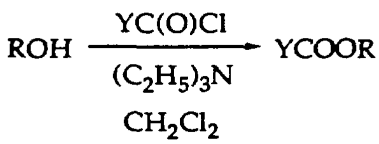


dans lequel Y et R sont définis comme à la revendication 1.

Les énol-esters d'aldéhydes et cétones odorants ont également été préparés par des méthodes conventionnelles, à partir desdits aldéhydes et cétones, à l'aide de réactions du type de celles représentées ci-après :

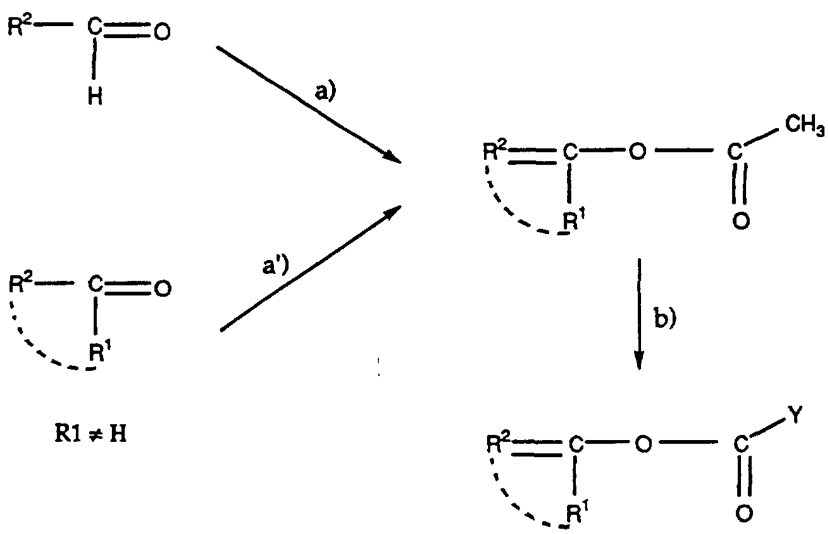


$R^1$, $R^2$ et Y sont définis comme à la revendication 1b.

a) anhydride acétique; acétate de potassium; éthylamine ; 120° ; voir, par exemple, D.P. Simmons et al., Helv. Chim. Acta 71, 1000 (1988)
a') anhydride acétique ; acide p-toluènesulfonique (cat.) ; 120° ; voir, par exemple, T. Taapken et al., J. Chem. Soc. Perkin Trans. I, 1994, 1439
b) tert-butoxyde de potassium ; YCOCl ; voir, par exemple, P. Duhamel et al., J. Chem. Soc. Perkin Trans. I, 1993 2509

Les conditions de réaction sont décrites en détail dans les exemples de préparation présentés ci-après, dans lesquels les températures sont en degrés centigrades et les abréviations ont le sens usuel dans l'art. L'invention sera également illustrée à l'aide d'exemples d'application.

## Manières de réaliser l'invention

Exemple 1

Préparation de mono-esters

### Méthode générale

A une solution agitée de l'alcool ROH approprié (0,064 mole) et triéthylamine (7,4 g, 0,073 mole) dans $CH_2Cl_2$ (110 ml), on a ajouté goutte-à-goutte pendant 20 min à 15° sous $N_2$ le chlorure d'acyle YC(O)Cl correspondant (0,07 mole), où Y est un radical alkyle de $C_7$ à $C_{24}$, linéaire ou ramifié, saturé ou insaturé. Après 2 h à température ambiante, le mélange a été versé sur $NaHCO_3$ aq. sat. (excès) et la phase organique a été séparée. L'extraction de la phase aqueuse avec $CH_2Cl_2$ a été suivie du lavage des phases organiques combinées avec NaCl aqueux à 10%. Le produit de la réaction a été séché sur $Na_2SO_4$, concentré et distillé, pour fournir l'ester désiré à l'état pur et avec 80 à 90% de rendement.

Les mono-esters suivants ont été préparés selon la méthode générale décrite ci-dessus.

a. octanoate de 2-phényléthyle

P. éb. 100-101°/4 Pa
IR($CHCl_3$) : 2930, 2858, 1728, 1498, 1455, 1168, 1106 $cm^{-1}$
RMN($^1H$, 360MHz) : 0,88(t, J=7Hz, 3H) ; 1,27(8H) ; 1,59(2H) ; 2,28(t, J=7Hz ,2H) ; 2,93(t, J=7Hz, 2H) ; 4,29 (t, J=7Hz, 2H) 7,20-7,35(5H) δ ppm.
RMN($^{13}C$) : 173,7(s) ; 138,0(s) ; 128,9(d) ; 128,5(d) ; 126,5(d) ; 64,7(t) ; 35,2(t) ; 34,4(t) ; 31,7(t) ; 29,1(t) ; 28,9 (t) ; 25,0(t) ; 22,6(t) ; 14,0(q) δ ppm.
SM : 248(0, $M^+$), 127(2), 104(100), 57(12).

b. hexadécanoate de 2-phényléthyle

P. f. 40°
IR($CHCl_3$) : 2927, 2855, 1728, 1456, 1174 $cm^{-1}$
RMN($^1H$, 360MHz) : 0,88(t, J=7Hz, 3H) ; 1,26(24H) ; 1,58(2H) ; 2,27(t, J=7Hz, 2H) ; 2,93(t, J=7Hz, 2H) ; 4,29 (t, J=7Hz, 2H) ; 7,20-7,35(5H) δ ppm.
RMN($^{13}C$): 173,7(s) ; 138,0(s) ; 128,9(d) ; 128,5(d) ; 126,5(d) ; 64,7(t) ; 35,3(t) ; 34,4(t) ; 32,0(t) ; 29,7(2t) ; 29,5(t) ; 29,4(t) ; 29,3(t) ; 29,2(t) ; 25,0(t) ; 22,7(t) ; 14,1(q) δ ppm.
SM : 360(0, $M^+$), 104(100).

c. octanoate de 3,7-diméthyl-oct-6-ényle

P. éb. 108-109°/2,7 Pa
IR($CHCl_3$): 2829, 2858, 1725, 1460, 1379, 1231, 1171, 1106 $cm^{-1}$
RMN($^1H$, 360MHz) : 0,88(t, J=7Hz, 3H) ; 0,91(d, J=7Hz, 3H) ; 1,10-1,80(15H) ; 1,61(s, 3H) ; 1,68(s, 3H) ; 1,98 (m, 2H) ; 2,29(t, J=7Hz, 2H) ; 4,10(m, 2H) ; 5,09(large t, J=7Hz, 1H) δ ppm.
RMN($^{13}C$) : 173,9(s) ; 131,3(s) ; 124,7(d) ; 62,8(t) ; 37,1(t) ; 35,6(t) ; 34,5(t) ; 31,7(t) ; 29,6(d) ; 29,2(t) ; 29,0 (t) ; 25,7(q) ; 25,5(t) ; 25,1(t) ; 22,6(t) ; 19,5(q) ; 17,7(q) ; 14,1(q) δ ppm.
SM : 282(0, $M^+$), 138(30), 123(56), 109(25), 95(77), 81(100), 69(59).

d. hexadécanoate de 3,7-diméthyl-oct-6-ényle

P. éb. (four à boules) 210-230°/5,3 Pa
IR($CHCl_3$) : 2827, 2855, 1725, 1465, 1233, 1178 $cm^{-1}$
RMN($^1H$, 360MHz) : 0,88(t, J=7Hz, 3H) ; 0,92(d, J=7Hz, 3H) ; 1,15-1,35(26H) ; 1,44(m, 1H) ; 1,60(s, 3H) ; 1,68 (s, 3H) ; 1,50-1,70(4H) ; 1,98(m, 2H) ; 2,28(t, J=7Hz, 2H) ; 4,10(m, 2H) ; 5,09(large t, J=7FIz, 1H) δ ppm.
RMN($^{13}C$) : 173,9(s) ; 131,3(s) ; 124,6(d) ; 62,8(t) ; 37,1(t) ; 35,6(t) ; 34,5(t) ; 32,0(t) ; 29,7(2t) ; 29,6(d) ; 29,5(t) ; 29,4(t) ; 29,3(t) ; 29,2(t) ; 25,7(q) ; 25,5(t) ; 25,1(t) ; 22,7(t) ; 19,5(q) ; 17,7(q) ; 14,1(q) δ ppm.
SM : 394(0, $M^+$), 138(32), 123(30), 95(60), 82(70), 69(70), 57(100).

e. octanoate de 3-méthyl-5-phénylpentyle

P. éb. 133-135°/2,7 Pa
IR($CHCl_3$) : 2930, 2858, 1728, 1496, 1456, 1231, 1171, 1106 $cm^{-1}$
RMN($^1H$, 360MHz) : 0,88(t, J=7Hz, 3H) ; 0,98(d, J=7Hz, 3H) ; 1,28(8H) ; 1,40-1,80(7H) ; 2,27(t, J=7Hz, 2H) ; 2,62(m, 2H) ; 4,11(m, 2H) ; 7,10-7/30(5H) δ ppm.
RMN($^{13}C$) : 173,9(s) ; 142,7(s) ; 128,3(2d) ; 125,7(d) ; 62,6(t) ; 38,8(t) ; 35,5(t) ; 34,4(t) ; 33,3(t) ; 31,7(t) ; 29,6 (d) ; 29,2(t) ; 29,0(t) ; 25,0(t) ; 22,6(t) ; 19,5(q) ; 14,1 (q) δ ppm.
SM : 304(0, $M^+$), 160(55), 131(30), 104(100), 91(56).

f. hexadécanoate de 3-méthyl-5-phénylpentyle

P. éb. (four à boules) 250°/4 Pa
IR($CHCl_3$) : 2927, 2855, 1727, 1456, 1235, 1178 $cm^{-1}$
RMN($^1H$, 360MHz) : 0,88(t, J=7Hz, 3H) ; 0,97(d, J=7Hz, 3H) ; 1,26(24H) ; 1,48(2H) ; 1,55-1,80(5H) ; 2,27(t, J=7Hz, 2H) ; 2,62(m, 2H) ; 4,10(m, 2H) ; 7,15-7,30(5H) δ ppm.
RMN($^{13}C$) : 173,9(s) ; 142,6(s) ; 128,3(2d) ; 125,7(d) ; 62,6(t) ; 38,8(t) ; 35,5(t) ; 34,5(t) ; 33,3(t) ; 32,0(t) ; 29,7 (2t) ; 29,7(d) ; 29,6(t) ; 29,5(t) ; 29,4(t) ; 29,3(t) ; 29,2(t) ; 25,1(t) ; 22,7(t) ; 19,5(q) ; 14,1(q) δ ppm.
SM : 416(0, $M^+$), 160(43), 129(22), 115(36), 104(72), 91(68), 71(61), 57(100).

g. octanoate de 7-p-menthanyle (cis/trans 70:30)

P. éb. 105-115°/2,7Pa
IR($CHCl_3$) : 2929, 2858, 1724, 1453, 1232, 1172, 1106 $cm^{-1}$
RMN($^1H$, 360MHz) : cis-7 : 0,86(d, J=7Hz, 6H) ; 0,88(t, J=7Hz, 6H) ; 2,30(t, J=7Hz, 2H) ; 4,02(d, J=7Hz, 2H) δ ppm. trans-7 : 2,30(t, J=7Hz, 2H) ; 3,88(d, J=7Hz, 2H) δ ppm.
RMN($^{13}C$): cis-7 : 174,0(s) ; 66,6(t) ; 43,0(d) ; 34,5(t) ; 33,9(d) ; 30,6(d) ; 29,9(t) ; 29,2(t) ; 29,1(t) ; 26,5(t) ; 25,6 (t) ; 25,1(t) ; 20,3(q) ; 14,0(q) δ ppm. trans-7 : 174,0(s) ; 69,5(t) ; 44,1(d) ; 37,5(d) ; 32,9(d) ; 31,7(t) ; 22,6(t) ; 19,8(q) ; 14,0(q) 8 ppm.
SM : cis-7 : 282(0, $M^+$), 138(23), 123(17), 109(35), 95(100), 81(27). trans-7: 282(0, $M^+$), 138(27), 123(17), 109(22), 95(100), 81(35).

h. octanoate de déc-9-ényle

P. éb. 120-121°/2,7 Pa
IR($CHCl_3$) : 2830, 2857 1725, 1466, 1171 $cm^{-1}$
RMN($^1H$, 360MHz) : 0,88(t, J=7Hz, 3H) ; 1,30(18H) ; 1,61(4H) ; 2,04(large q, J=7Hz, 2H) ; 2,29(t, J=7Hz, 2H) ; 4,06(t, J=7Hz, 2H) ; 4,93(large d, J=11Hz, 1H) ; 4,99(large d, J=17Hz, 1H) ; 5,80(m, 1H) δ ppm.
RMN($^{13}C$) : 173,9(s) ; 139,1(d) ; 114,2(t) ; 64,4(t) ; 34,5(t) ; 33,8(t) ; 31,8(t) ; 29,4(t) ; 29,3(t) ; 29,2(t) ; 29,1(t) ; 29,0(2t) ; 28,8(t) ; 26,0(t) ; 25,1(t) ; 22,7(t) ; 14,1(q) δ ppm.
SM : 282(0, $M^+$), 145(38), 109(38), 96(86), 82(89), 68(91), 55(100).

i. nonanoate de déc-9-ényle

P. éb. (four à boules) 130-160°/2,7 Pa
IR($CHCl_3$) : $cm^{-1}$
RMN($^1H$, 360MHz) : 0,88(t, J=7Hz, 3H) ; 1,20-1,45(20H) ; 1,61(4H) ; 2,04 (large q, J=7Hz, 2H) ; 2,29(t, J=7Hz, 2H) ; 4,06(t, J=7Hz, 2H) ; 4,93(large d, J=11Hz, 1H) ; 4,99(large d, J=17Hz, 1H) ; 5,80(m, 1H) 8 ppm.
RMN($^{13}C$) : 174,0(s) ; 139,1(d) ; 114,2(t) ; 64,4(t) ; 34,5(t) ; 33,8(t) ; 31,9(t) ; 29,4(t) ; 29,3(t) ; 29,2(2t) ; 29,1 (t) ; 29,0(t) ; 28,7(t) ; 26,0(t) ; 25,1(t) ; 22,7(t) ; 14,1(q) δ ppm.
SM : 296(0, $M^+$), 159(27), 138(28), 96(100), 82(75), 68(73), 55(83).

j. décanoate de déc-9-ényle

P. éb. 144-145°/2,7 Pa

$IR(CHCl_3)$ : 2929, 2856 1726, 1466, 1178 $cm^{-1}$
RMN($^1H$, 360MHz) : 0,88(t, J=7Hz, 3H) ; 1,30(22H) ; 1,61(4H) ; 2,04(large q, J=7Hz, 2H) ; 2,29(t, J=7Hz, 2H) ; 4,05(t, J=7Hz, 2H) ; 4,93(large d, J=11Hz, 1H) ; 4,98(large d, J=17Hz, 1H) ; 5,80(m, 1H) δ ppm.
RMN($^{13}C$) : 173,9(s) ; 139,1(d) ; 114,2(t) ; 64,4(t) ; 34,5(t) ; 33,8(t) ; 31,9(t) ; 29,5(t) ; 29,3(t) ; 29,2(t) ; 29,1(t) ; 29,0(t) ; 28,7(t) ; 26,0(t) ; 25,1(t) ; 22,7(t) ; 14,1(q) δ ppm.
SM : 310(0, $M^+$), 138(33), 109(29), 96(100), 82(82), 68(98).

k. undécanoate de déc-9-ényle

P. éb. (four à boules) 130-150°/20 Pa
$IR(CHCl_3)$ : $cm^{-1}$
RMN($^1H$, 360MHz): 0,88(t, J=7Hz, 3H) ; 1,20-1,45(24H) ; 1,61(4H) ; 2,04(large q, J=7Hz, 2H) ; 2,29(t, J=7Hz, 2H) ; 4,06(t, J=7Hz, 2H) ; 4,93(large d, J=11Hz, 1H) ; 4,99(large d, J=17Hz, 1H); 5,81(m, 1H) δ ppm.
RMN($^{13}C$) : 173,9(s) ; 139,1(d) ; 114,2(t) ; 64,4(t) ; 34,5(t) ; 33,8(t) ; 32,0(t) ; 29,6(2t) ; 29,4(2t) ; 29,3(t) ; 29,1 (t) ; 29,0(t) ; 28,8(t) ; 26,0(t) ; 25,1(t) ; 22,7(t) ; 14,1(q) δ ppm.
SM : 324(0, $M^+$), 187(20), 138(43), 109(33), 96(100), 82(96), 68(89), 55(91).

l. dodécanoate de déc-9-ényle

P. éb. 164-165°/4 Pa
$IR(CHCl_3)$: 2928, 2856 1726, 1216 $cm^{-1}$
RMN($^1H$, 360MHz) : 0,89(t, J=7Hz, 3H) ; 1,28(26H) ; 1,62(4H) ; 2,04(large q, J=7Hz, 2H) ; 2,29(t, J=7Hz, 2H) ; 4,06(t, J=7Hz, 2H) ; 4,93(large d, J=11Hz, 1H) ; 4,98(large d, J=17Hz, 1H) ; 5,80(m, 1H) δ ppm.
RMN($^{13}C$) : 174,0(s) ; 139,2(d) ; 114,2(t) ; 64,4(t) ; 34,5(t) ; 33,8(t) ; 32,0(t) ; 29,6(t) ; 29,5(t) ; 29,4(2t) ; 29,3 (t) ; 29,2(t) ; 29,1(2t) ; 28,9(t) ; 28,7(t) ; 26,0(t) ; 25,1(t) ; 22,7(t) ; 14,1(q) δ ppm.
SM : 338(0, $M^+$), 138(48), 109(35), 96(100), 82(96), 68(89), 55(98).

m. tridécanoate de déc-9-ényle

P. éb. (four à boules) 150-175°/13 Pa
$IR(CHCl_3)$ : $cm^{-1}$
RMN($^1H$, 360MHz): 0,88(t, J=7Hz, 3H) ; 1,20-1,45(28H) ; 1,61(4H) ; 2,04(large q, J=7Hz, 2H) ; 2,29(t, J=7Hz, 2H) ; 4,05(t, J=7Hz, 2H) ; 4,93(large d, J=11Hz, 1H) ; 4,99(large d, J=17Hz, 1H) ; 5,81(m, 1H) δ ppm.
RMN($^{13}C$) : 174,0(s) ; 139,1(d) ; 114,2(t) ; 64,4(t) ; 34,5(t) ; 33,8(t) ; 32,0(t) ; 29,7(t) ; 29,5(t) ; 29,4(t) ; 29,3(t) ; 29,2(t) ; 29,1(t) ; 29,0(t) ; 28,7(t) ; 26,0(t) ; 25,1(t) ; 22,7(t) ; 14,1(q) δ ppm.
SM : 352(0, $M^+$), 138(52), 110(41), 96(99), 82(83), 68(93), 55(100).

n. tétradécanoate de déc-9-ényle

P. éb. (four à boules) 200-220°/4 Pa
$IR(CHCl_3)$ : $cm^{-1}$
RMN($^1H$, 360MHz) : 0,88(t, J=7Hz, 3H) ; 1,28(30H) ; 1,62(4H) ; 2,04(large q, J=7Hz, 2H) ; 2,29(t, J=7Hz, 2H) ; 4,06(t, J=7Hz, 2H) ; 4,93(large d, J=11Hz, 1H) ; 4,99(large d, J=17Hz, 1H) ; 5,81(m, 1H) δ ppm.
RMN($^{13}C$) : 174,0(s) ; 139,1(d) ; 114,2(t) ; 64,4(t) ; 34,5(t) ; 34,5(t) ; 33,8(t) ; 32,0(t) ; 29,7(t) ; 29,5(t) ; 29,4(t) ; 29,3(2t) ; 29,1(t) ; 29,0(t) ; 28,7(t) ; 26,0(t) ; 25,1(t) ; 22,7(t) ; 14,1(q) δ ppm.
SM : 366(0, $M^+$), 138(52), 110(47), 96(100), 82(96), 55(96).

o. pentadécanoate de déc-9-ényle

P. éb. (four à boules) 175-210°/13 Pa
$IR(CHCl_3)$ : $cm^{-1}$
RMN($^1H$, 360MHz) : 0,88(t, J=7Hz, 3H) ; 1,20-1,45(32H) ; 1,62(4H) ; 2,04(large q, J=7Hz, 2H) ; 2,29(t, J=7Hz, 2H) ; 4,06(t, J=7Hz, 2H) ; 4,93(large d, J=11Hz, 1H) ; 4,99(large d, J=17Hz, 1H) ; 5,81(m, 1H) δ ppm.
RMN($^{13}C$) : 174,0(s) ; 139,1(d) ; 114,2(t) ; 64,4(t) ; 34,5(t) ; 33,8(t) ; 32,0(t) ; 29,7(2t) ; 29,5(t) ; 29,4(t) ; 29,3 (t) ; 29,2(t) ; 29,1(t) ; 29,0(t) ; 28,7(t) ; 26,0(t) ; 25,1(t) ; 22,7(t) ; 14,1(q) δ ppm.
SM : 380(0, $M^+$), 138(50), 110(30), 96(100), 82(85), 68(79), 55(80).

p. hexadécanoate de déc-9-ényle

RMN($^1$H, 360MHz) : 5,84(m, 1H) ; 4,95(m, 2H) ; 4,05(t, 2H) ; 3,73(q, 4H) ; 2,31(t, 2H) ; 2,05(large q, 2H) ; 1,61 (m, 4H) ; 1,2-1,4(large m, 30H) ; 0,89(m, 3H) δ ppm.
RMN($^{13}$C) : 14,12(q) ; 18,44(q) ; 22,72(t) ; 25,06(t) ; 25,95(t) ; 28,68(t) ; 28,92(t) ; 29,05(t) ; 29,21(t) ; 29,30(t) ; 29,39(t) ; 29,51(t) ; 29,64(t) ; 29,71(t) ; 31,95(t) ; 33,80(t) ; 34,45(t) ; 58,45(t) ; 64,41(t) ; 114,17(t) ; 139,16(d) ; 174,07(s) δ ppm.
SM : 394(0, M$^+$), 96(72), 83(70), 82(88), 55(100).

q. hexadécanoate de 1-méthylpentyle

RMN($^1$H, 360MHz) : 4,90(m, 1H) ; 2,26(t, 2H) ; 1,65(m, 4H) ; 1,25-1,32(m, 28H) ; 1,20(d, 3H) ; 0,89(q, 6H) δ ppm.
RMN($^{13}$C) : 13,97(q) ; 14,00(q) ; 20,40(q) ; 22,56(t) ; 22,7(t) ; 25,1(t) ; 27,6(t) ; 29,2(t) ; 29,3(t) ; 29,4(t) ; 29,5 (t) ; 29,6(t) ; 29,7(t) ; 29,73(t) ; 31,97(t) ; 34,83(t) ; 35,75(t) ; 70,74(d) ; 173,50(s) δ ppm.
SM : 84(100), 69(50), 57(70), 55(62), 43(98).

r. hexadécanoate de 7-p-menthanyle

RMN($^1$H, 360MHz) : 0,80-0,95(m, 9H) ; 1,2-2,0(large m, 37H) ; 2,32(t, 2H) ; 3,88 et 4,02(d, 2H) δ ppm.
RMN($^{13}$C) : 14,09(t) ; 19,8(t) ; 20,2(t) ; 22,7(t) ; 25,12(t) ; 22,56(t) ; 26,44(t) ; 29,10(t) ; 29,22(t) ; 29,30(t) ; 29,38(t) ; 29,51(t) ; 29,63(t) ; 29,71(t) ; 29,92(t) ; 30,54(d) ; 31,97(t) ; 32,90(d) ; 33,90(d) ; 34,50(t) ; 37,50(d) ; 42,98(d) ; 44,11(d) ; 66,62(t) ; 69,50(t) ; 174,05(s) δ ppm.
SM : 93(70), 77(33), 69(100), 43(48), 41(92).

s. hexadécanoate d'hexyle

RMN($^1$H, 360MHz) : 4,02(t, 2H) ; 3,86(large q, 2H) ; 2,29(t, 2H) ; 1,60(large m, 4H) ; 1,2-1,4(large m, 28H) ; 0,90(m, 6H) δ ppm.
RMN($^{13}$C) : 14,1(q) ; 13,97(q) ; 22,58(t) ; 22,74(t) ; 25,11(t) ; 25,68(t) ; 28,75(t) ; 29,24(t) ; 29,34(t) ; 29,41(t) ; 29,54(t) ; 29,67(t) ; 29,72(t) ; 29,74(t) ; 31,51(t) ; 32,00(t) ; 34,50(t) ; 58,42(t) ; 64,46(t) ; 174,07(s) δ ppm.
SM : 340(2, M$^+$), 84(100), 57(55), 56(58), 43(80).

t. hexadécanoate de (Z)-hex-3-ényle

RMN($^1$H, 360MHz) : 5,45(m, 1H) ; 5,26(m, 1H) ; 4(t, 2H) ; 3,42(s, 4H) ; 2,2-3,3(m, 8H) ; 2,0(m, 1H) ; 1,6(m, 8H) ; 1,2(large m, 30H) ; 0,8-1,0(m, 6H) δ ppm.
RMN($^{13}$C) : 14,14(q) ; 14,24(q) ; 20,63(t) ; 22,73(t) ; 25,02(t) ; 25,59(t) ; 26,37(t) ; 26,72(t) ; 26,82(t) ; 29,20(t) ; 29,32(t) ; 29,40(t) ; 29,51(t) ; 29,73(t) ; 31,97(t) ; 34,39(t) ; 93,77(t) ; 123,83(d) ; 134,50(d) ; 173,93(s) δ ppm.
SM : 83(25), 82(100), 67(38), 55(27), 43(14).

u. hexadécanoate de (E)-3,7-diméthyl-octa-2,6-diényle

RMN($^1$H, 360MHz) : 0,87(t, 3H) ; 1,26(m, 26H) ; 1,6(s, 3H) ; 1,68(s, 3H) ; 1,70(s, 3H) ; 2,0-2,15(m, 4H) ; 2,30 (t, 2H) ; 4,6(d, 2H) ; 5,09(t, 1H); 5,35(t, 1H) δ ppm.
RMN($^{13}$C) : 14,12(q) ; 16,48(q) ; 17,69(q) ; 27,73(t) ; 25,08(t) ; 25,68(t) ; 26,38(t) ; 29,22(t) ; 29,33(t) ; 29,41 (t) ; 29,53(t) ; 29,67(t) ; 29,72(t) ; 31,99(t) ; 34,45(t) ; 35,59(t) ; 61,19(t) ; 116,60(d) ; 123,85(d) ; 131,80(s) ; 142,06(s) ; 173,91(s) δ ppm.
SM : 392(0, M$^+$), 93(94), 69(100), 43(89), 41(70).

Exemple 2

Préparation de di-esters

**Méthode générale**

On a utilisé la méthode décrite dans l'Exemple 1, mais en employant la moitié des quantités molaires du dichlorure de diacyle correspondant $[(CH_2)_n[C(O)Cl]_2$, n=0 à 6].

Les di-esters suivants ont été préparés selon cette méthode générale :

a. oxalate de bis(déc-9-ényl)

P. éb. (four à boules): 200°/40 Pa
IR($CHCl_3$) : 2930, 2860, 1770, 1740, 1460, 1312, 1175, 912 $cm^{-1}$
RMN($^1H$, 360MHz) : 1,25-1,45(20H) ; 1,74(m, 4H) ; 2,04(large q, J=7Hz, 4H) ; 4,28(t, J=7Hz, 4H) ; 4,93(large d, J=11Hz, 2H) ; 4,99(large d, J=17Hz, 2H) ; 5,80(m, 2H) δ ppm.
RMN($^{13}C$) : 158,1(s) ; 139,0(d) ; 114,2(t) ; 67,1(t) ; 33,8(t) ; 29,3(t) ; 29,1(t) ; 29,0(t) ; 28,9(t) ; 28,3(t) ; 25,7(t) δ ppm.
SM : 366(0, $M^+$), 138(9), 109(13), 96(27), 83(55), 69(45), 55(100).

b. malonate de bis(déc-9-ényl)

P. éb. (four à boules): 210°/40 Pa
IR($CHCl_3$) : 2940, 2862, 1740, 1465, 1336, 1276, 1155, 915 $cm^{-1}$
RMN($^1H$, 360MHz) : 1,25-1,45(20H) ; 1,64(m, 4H) ; 2,04(large q, J=7Hz, 4H) ; 3,37(s, 2H) ; 4,14(t, J=7Hz, 4H) ; 4,93(large d, J=11Hz, 2H) ; 4,99 (large d, J=17Hz, 2H) ; 5,81 (m, 2H) δ ppm.
RMN($^{13}C$) : 166,6(s) ; 139,1(d) ; 114,2(t) ; 65,6(t) ; 41,7(t) ; 33,8(t) ; 29,4(t) ; 29,2(t) ; 29,1(t) ; 28,9(t) ; 28,5(t) ; 25,8(t) δ ppm.
SM : 380(0, $M^+$), 138(25), 109(21), 105(42), 96(60), 83(100), 68(65), 55(95).

c. butanedioate de bis(déc-9-ényl)

IR($CHCl_3$) : 2935, 2860, 1735, 1460, 1160, 995, 910 $cm^{-1}$
RMN($^1H$, 360MHz) : 1,25-1,45(20H) ; 1,63(m, 4H) ; 2,04(large q, J=7Hz, 4H) ; 2,62(s, 4H) ; 4,08(t, J=7Hz, 4H) ; 4,93(large d, J=11Hz, 2H) ; 4,99(large d, J=17Hz, 2H) ; 5,81(m, 2H) δ ppm.
RMN($^{13}C$) : 172,3(s) ; 139,1(d) ; 114,2(t) ; 64,9(t) ; 33,8(t) ; 29,4(t) ; 29,3(t) ; 29,2(t) ; 29,1(t) ; 28,9(t) ; 28,7(t) ; 25,9(t) δ ppm.
SM : 394(0, $M^+$), 138(10), 119(22), 101(60), 97(38), 83(100), 69(44), 55(76).

d. pentanedioate de bis(déc-9-ényl)

IR($CHCl_3$) : 2940, 2880, 1740, 1464, 1180, 1000, 918 $cm^{-1}$
RMN($^1H$, 360MHz) : 1,25-1,45(20H); 1,62(m, 4H) ; 1,95(t, J=7, 7Hz, 2H) ; 2,04(large q, J=7Hz, 4H) ; 2,37(t, J=7Hz, 4H) ; 4,06(t, J=7Hz, 4H) ; 4,93(large d, J=11Hz, 2H) ; 4,99(large d, J=17Hz, 2H) ; 5,81(m, 2H) δ ppm.
RMN($^{13}C$) : 173,0(s) ; 139,1(d) ; 114,2(t) ; 64,6(t) ; 33,8(t) ; 33,4(t) ; 29,4(t) ; 29,2(t) ; 29,1(t) ; 28,9(t) ; 28,7(t) ; 25,9(t) δ ppm.
SM : 408(0, $M^+$), 115(100), 97(14), 87(20), 83(26), 69(19), 55(39).

e. hexanedioate de bis(déc-9-ényl)

IR($CHCl_3$) : 2942, 2864, 1740, 1466, 1180, 1000, 918 $cm^{-1}$
RMN($^1H$, 360MHz) : 1,25-1,45(20H) ; 1,55-1,75(8H) ; 2,04(large q, J=7Hz, 4H) ; 2,32(m, 4H) ; 4,06(t, J=7Hz, 4H) ; 4,93(large d, J=11Hz, 2H) ; 4,99(large d, J=17Hz, 2H) ; 5,81(m, 2H) δ ppm.
RMN($^{13}C$) : 173,4(s) ; 139,1(d) ; 114,2(t) ; 64,5(t) ; 34,0(t) ; 33,8(t) ; 29,4(t) ; 29,2(t) ; 29,0(t) ; 28,9(t) ; 28,7(t) ; 25,9(t) ; 24,5(t) δ ppm.
SM : 422(0, $M^+$), 129(90), 111(64), 101(31), 95(23), 83(60), 67(41), 55(100).

f. oxalate de (E,E)-bis(3,7-diméthyl-octa-2,6-diényl)

IR($CHCl_3$) : 2929, 1740, 1449, 1378, 1302, 1168 $cm^{-1}$
RMN($^1H$, 360MHz) : 1,60(s, 6H) ; 1,68(s, 6H) ; 1,75(s, 6H) ; 2,09(8H) ; 4,80(d, J=7Hz, 4H) ; 5,07(m, 2H) ; 5,41 (large t, J=7Hz, 2H) δ ppm.
RMN($^{13}C$) : 158,0(s) ; 144,3(s) ; 132,0(s) ; 123,6(d) 116,9(d) ; 63,8(t) ; 39,6(t) ; 26,2(t) ; 25,7(q) ; 17,7(q) ; 16,6 (q) δ ppm.
SM : 362(0, $M^+$), 135(7), 93(20), 81(29), 69(100).

g. malonate de (E,E)-bis(3,7-diméthyl-octa-2,6-diényl)

IR($CHCl_3$) : 2930, 1728, 1447, 1379, 1278, 1149, 983 $cm^{-1}$
RMN($^1H$, 360MHz) : 1,60(s, 6H) ; 1,69(s, 6H) ; 1,71(s, 6H) ; 2,08(8H) ; 3,38(s, 2H) ; 4,65(d, J=7Hz, 4H) ; 5,08 (m, 2H) ; 5,34(large t, J=7Hz, 2H) δ ppm.
RMN($^{13}C$): 166,6(s) ; 142,9(s) ; 131,9(s) ; 123,7(d) ; 117,8(d) ; 62,4(t) ; 41,7(t) ; 39,6(t) ; 26,4(t) ; 25,7(q) ; 17,7 (q) ; 16,5(q) δ ppm.
SM : 376(0, $M^+$), 136(17), 121(15), 93(39), 81(33), 69(100).

h. butanedioate de (E,E)-bis(3,7-diméthyl-octa-2,6-diényl)

IR($CHCl_3$) : 2930, 1729, 1446, 1384, 1231, 1162 $cm^{-1}$
RMN($^1H$, 360MHz) : 1,61(s, 6H) ; 1,69(s, 6H) ; 1,70(s, 6H) ; 2,08(8H) ; 2,64(s, 414) ; 4,62(d, J=7Hz, 4H) ; 5,08 (m, 2H) ; 5,34(large t, J=7Hz, 2H) δ ppm.
RMN($^{13}C$): 172,3(s) ; 142,3(s) ; 131,8(s) ; 123,8(d) ; 118,3(d) ; 61,7(t) ; 39,6(t) ; 29,3(t) ; 26,4(t) ; 25,7(q) ; 17,7 (q) ; 16,5(q) δ ppm.
SM : 390(0, $M^+$), 136(17), 121(19), 93(62), 81(27), 69(100).

i. pentanedioate de (E,E)-bis(3,7-diméthyl-octa-2,6-diényl)

IR($CHCl_3$) : 2930, 1727, 1450, 1232, 1176 $cm^{-1}$
RMN($^1H$, 360MHz) : 1,61(s, 6H) ; 1,68(s, 6H) ; 1,70(s, 6H) ; 1,96(m, 2H) ; 2,07(8H) ; 2,37(t, J=7Hz, 4H) ; 4,59 (d, J=7Hz, 4H) ; 5,08(m, 2H) ; 5,33(large t, J=7Hz, 2H) δ ppm.
RMN($^{13}C$) : 172,9(s) ; 142,2(s) ; 131,8(s) ; 123,8(d) ; 118,4(d) ; 61,4(t) ; 39,6(t) ; 33,4(t) ; 26,3(t) ; 25,7(q) ; 20,3 (t) ; 17,7(q) ; 16,5(q) δ ppm.
SM : 404(0, $M^+$), 136(20), 121(18), 93(55), 81(48), 69(100).

j. hexanedioate de (E,E)-bis(3,7-diméthyl-octa-2,6-diényl)

IR($CHCl_3$) : 2931, 1727, 1446, 1384, 1233, 1174 $cm^{-1}$
RMN($^1H$, 360MHz) : 1,60(s, 6H) ; 1,68(4H) ; 1,68(s, 6H) ; 1,70(6H) ; 2,07(8H) ; 2,33(4H) ; 4,59(d, J=7Hz, 4H) ; 5,08(m, 2H) ; 5,33(large t, J=7Hz, 2H) δ ppm
RMN($^{13}C$) : 173,3(s) ; 142,2(s) ; 131,8(s) ; 123,8(d) ; 118,4(d) ; 61,3(t) ; 39,8(t) ; 34,0(t) ; 26,4(t) ; 25,7(q) ; 24,5 (t) ; 17,7(q) ; 16,5(q) δ ppm.
SM : 418(0, $M^+$), 135(15), 121(15), 93(52), 81(32), 69(100).

Exemple 3

Préparation d'énol-esters

**Méthode générale**

Dans un ballon tricol de 500 ml, sous argon, on a placé 0,162 mole de l'aldéhyde de formule

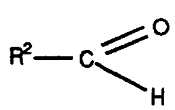

approprié, 2,5 g (26 mmole) d'acétate de potassium anydre, 34,45 g (0,34 mole) de triéthylamine et 250 ml d'anhydride acétique. On a chauffé 6 h à 120°, refroidit à température ambiante, versé sur glace, extrait 3 fois à l'éther de pétrole 30-50°, lavé 6 fois avec 100 ml de $NaHCO_3$ saturé, puis à l'eau jusqu'à neutralité. On a séché sur $Na_2SO_4$, filtré, concentré sous vide et distillé sur colonne Vigreux pour obtenir l'énolacétate correspondant à l'aldéhyde de départ, sous forme d'un mélange d'isomères E et Z. Ces derniers ont ensuite été séparés par chromatographie préparative en phase gazeuse.
Lorsqu'on est parti d'une cétone de formule

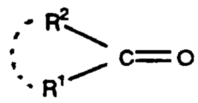

on a suivi une méthode analogue a celle décrite par T. Taapken et al., J. Chem. Soc. Perkin Trans. I, 1994, 1439, en dissolvant la cétone dans l'anhydride acétique et traitant avec l'acide p-toluènesulfonique en tant que catalyseur, en éliminant l'acide acétique formé, pour obtenir l'énolacétate correspondant.

Les énolacétates ainsi préparés sont ensuite transformés en énol-esters de formule (I) en procédant comme suit.

Dans un ballon tricol de 500 ml sous argon, on a chargé 0,05 mole d'énolacétate et 75 ml de tétrahydrofurane absolu. On a refroidit à -60/-70° (bain neige carbonique/acétone) et introduit goutte à goutte une solution de 6,16 g (0,055 mole) de t-BuOK dans 60 ml de THF absolu (légèrement exothermique, le mélange réactionnel devient jaune intense). On a laissé agiter 1 h à -70° et ajouté goutte à goutte 0,055 mole de chlorure d'acyle approprié en solution dans 25 ml de THF absolu. Après 2 h à -70°, on a retiré le bain de refroidissement et ajouté rapidement 60 ml de solution saturée de $NaHCO_3$ (la température monte rapidement à -10°). On a extrait 2 fois à l'éther sulfurique, lavé une fois à $NaHCO_3$ saturé, une fois à l'eau saturée de sel, séché ($Na_2SO_4$) et concentré sous vide. Le produit est alors purifié par chromatographie flash (diamètre colonne = 9 cm, hexane/éther 98:2) et, après réunion des fractions intéressantes, concentré à sec sous vide absolu.

Selon cette méthode on a préparé les énol-acétates et énol-esters suivants :

a. acétate de 2-(4-tert-butylbenzyl)-1-propényle

Isomère (Z)

RMN($^1$H, 360MHz, $CDCl_3$) : 1,32(s, 9H) ; 1,59(s, 3H) ; 2,16(s, 3H) ; 3,43(s, 2H) ; 6,99(large s, 1H) ; 7,10 (d, J=8, 2H) ; 7,31(d, J=8, 2H) δ ppm.

RMN($^{13}$C, 90MHz, $CDCl_3$) : 17,4(q) ; 20,8(q) ; 31,4(q) ; 31,4(q) ; 31,4(q) ; 35,3(t) ; 125,3(d) ; 125,3(d) ; 128,3(d); 128,3(d) ; 130,5(d) ; 34,4(s) ; 121,2(s) ; 136,0(s) ; 149,0(s) ; 168,3(s) δ ppm.

SM : 246(13, $M^+$); m/e, 204(17), 189(73), 171(7), 159(8), 147(31), 129(33), 119(100), 105(9), 91(40), 77 (8), 71(7), 57(60), 43(64).

Isomère (E)

RMN($^1$H, 360MHz, $CDCl_3$) : 1,32(s, 9H) ; 1,62(s, 3H) ; 2,15(s, 3H) ; 3,24(s, 2H) ; 7,05(large s, 1H) ; 7,12 (d, J=8, 2H) ; 7,31(d, J=8, 2H) δ ppm.

RMN($^{13}$C, 90MHz, $CDCl_3$) : 13,6(q) ; 20,8(q) ; 31,4(q) ; 31,4(q) ; 31,4(q) ; 39,8(t) ; 125,3(d); 125,3(d); 128,4(d) ; 128,4(d) ; 131,2(d) ; 34,4(s) ; 121,4(s); 135,9(s) ; 149,2(s) ; 168,3(s) δ ppm.

SM : 246(12, $M^+$); m/e, 204(41), 189(100), 171(3), 159(7), 147(42), 131(21), 119(57), 105(9), 91(32), 77 (6), 71(8), 57(45), 43(58).

b. acétate de 3,7-diméthyl-1,6-octadiényle

Les caractères analytiques de ce composé étaient identiques à ceux publiés dans la littérature (voir, D.P. Simmons et al., ref. citée).

c. octanoate de 3,7-diméthyl-1,6-octadiényle

Isomère (Z)

RMN($^1$H, 360MHz, $CDCl_3$) : 0,89(t, J=7, 3H) ; 0,99(d, J=7, 3H) ; 1,30(m, 10H) ; 1,59(s, 3H) ; 1,68(s, 3H) ; 1,94(m, 2H) ; 2,39(t, J=7, 2H) ; 2,68(m, 1H) ; 4,67(dd, $J_1$=6, $J_2$=10, 1H) ; 5,10(m, 1H) ; 7,00(d, J=6, 1H) δ ppm.

RMN($^{13}$C, 90MHz, $CDCl_3$) : 14,1(q) ; 17,6(q) ; 20,9(q) ; 25,7(q) ; 22,6(t) ; 24,8(t) ; 25,9(t) ; 28,9(t) ; 29,0 (t) ; 31,7(t) ; 34,2(t) ; 37,4(t) ; 29,4(d) ; 120,1(d) ; 124,5(d) ; 133,1(d) ; 131,3(s) ; 171,0(s) δ ppm.

SM : 280(0, $M^+$); m/e, 198(1), 182(2), 154(8), 136(35), 127(100), 121(30), 109(20), 93(12), 82(17), 69 (23), 57(96), 41(32).

Isomère (E)

RMN($^1H$, 360MHz, $CDCl_3$) : 0,89(t, J=7, 3H) ; 1,02(d, J=7, 3H) ; 1,30(m, 10H) ; 1,59(s, 3H) ; 1,68(s, 3H) ; 1,96(m, 2H) ; 2,15(m, 1H) ; 2,36(t, J=7, 2H) ; 5,07(m, 1H) ; 5,29(dd, $J_1$=8, $J_2$=12, 1H) ; 7,08(d, J=12, 1H) δ ppm.
RMN($^{13}C$, 90MHz, $CDCl_3$) : 14,1(q) ; 17,7(q) ; 20,9(q) ; 25,7(q) ; 22,6(t) ; 24,7(t) ; 25,7(t) ; 28,9(t) ; 29,1 (t) ; 31,6(t) ; 34,1(t) ; 37,2(t) ; 32,0(d) ; 120,4(d) ; 124,3(d) ; 134,6(d) ; 131,5(s) ; 171,2(s) δ ppm.
SM : 280(0, $M^+$); m/e, 198(1), 185(1), 154(7), 136(32), 127(93), 121(26), 109(19), 93(11), 82(15), 69(23), 57(100), 41(32).

d. acétate de 2-méthyl-1-undécényle

Isomère (Z)

RMN($^1H$, 360MHz, $CDCl_3$) : 0,88(t, J=7, 3H) ; 1,27(m, 12H) ; 1,39(m, 2H) ; 1,63(divisé s, 3H) ; 2,11(m, 2H) ; 2,13(s, 3H) ; 6,83(large s, 1H) δ ppm.
RMN($^{13}C$, 90MHz, $CDCl_3$) : 14,1(q) ; 17,4(q) ; 20,7(q) ; 22,7(t) ; 27,0(t) ; 29,3-29,6(t) ; 29,3-29,6(t) ; 29,3-29,6(t) ; 29,3-29,6(t) ; 29,3-29,6(t) ; 31,9(t) ; 120,0(d) ; 122,3(s) ; 168,3(s) δ ppm.
SM : 226(3, $M^+$); m/e, 184(33), 166(1), 141(2), 124(3), 110(6), 95(14), 82(15), 71(100), 57(13), 43(50).

Isomère (E)

RMN($^1H$, 360MHz, $CDCl_3$) : 0,88(t, J=7, 3H) ; 1,27(m, 12H) ; 1,40(m, 2H) ; 1,66(divisé s, 3H) ; 1,95(m, 2H) ; 2,13(s, 3H) ; 6,88(large s, 1H) δ ppm.
RMN($^{13}C$, 90MHz, $CDCl_3$) : 13,6(q) ; 14,1(q) ; 20,8(q) ; 22,7(t) ; 27,6(t) ; 29,2(t) ; 29,3(t) ; 29,5(t) ; 29,6(t) ; 31,9(t) ; 33,9(t) ; 130,2(d) ; 122,0(s) ; 168,3(s) δ ppm.
SM : 226(2, $M^+$); m/e, 184(25), 166(1), 141(2), 123(3), 110(5), 95(12), 81(18), 71(100), 58(9), 43(40).

e. octanoate de 2-méthyl-1-undécényle

Isomère (Z)

RMN($^1H$, 360MHz, $CDCl_3$) : 0,88(t, J=7, 6H) ; 1,27(m, 22H) ; 1,39(m, 2H) ; 1,52(divisé s, 3H) ; 2,10(t, J=7, 2H) ; 2,37(t, J=7, 2H) ; 6,85(large s, 1H) δ ppm.
RMN($^{13}C$, 90MHz, $CDCl_3$) : 14,0(q) ; 14,1(q) ; 17,4(q) ; 22,6(t) ; 22,7(t) ; 24,9(t) ; 27,1(t) ; 28,9-29,6(t) ; 28,9-29,6(t) ; 28,9-29,6(t) ; 28,9-29,6(t) ; 28,9-29,6(t) ; 28,9-29,6(t) ; 28,9-29,6(t) ; 31,7(t) ; 31,9(t) ; 34,2 (t) ; 129,9(d) ; 122,2(s) ; 171,1(s) δ ppm.
SM : 310(0, $M^+$); m/e, 184(10), 127(85), 109(10), 95(6), 81(10), 71(25), 57(100), 43(10).

Isomère (E)

RMN($^1H$, 360MHz, $CDCl_3$) : 0,88(t, J=7, 6H) ; 1,27(m, 22H) ; 1,40(m, 2H) ; 1,67(divisé s, 3H) ; 1,95(t, J=7, 2H) ; 2,39(t, J=7, 2H) ; 6,89(large s, 1H) δ ppm.
RMN($^{13}C$, 90MHz, $CDCl_3$) : 13,6(q) ; 14,0(q) ; 14,1(q) ; 22,6(t) ; 22,7(t) ; 24,9(t) ; 27,6(t) ; 28,9-29,6(t) ; 28,9-29,6(t) ; 28,9-29,6(t) ; 28,9-29,6(t) ; 28,9-29,6(t) ; 28,9-29,6(t) ; 31,7(t) ; 31,9(t) ; 34,0(t) ; 34,2(t) ; 130,1(d) ; 121,9(s) ; 171,1(s) δ ppm.
SM : 310(0, $M^+$); m/e, 184(10), 127(87), 109(10), 97(7), 81(13), 71(28), 57(100), 43(9).

f. acétate de 1-undécényle

Isomère (Z)

RMN($^1H$, 360MHz, $CDCl_3$) : 0,88(t, J=7, 3H) ; 1,28(m, 12H) ; 1,37(m, 2H) ; 2,12(m, 2H) ; 2,14(s, 3H) ; 4,87 (dt, $J_1$=$J_2$=6, 1H) ; 6,99(d, J=6, 1H) δ ppm.
RMN($^{13}C$, 90MHz, $CDCl_3$) : 14,1(q) ; 20,8(q) ; 22,7(t) ; 24,4(t) ; 29,2-29,6(t) ; 29,2-29,6(t) ; 29,2-29,6(t) ; 29,2-29,6(t) ; 29,2-29,6(t) ; 31,9(t) ; 114,4(d) ; 134,0(d) ; 168,2(s) δ ppm.
SM : 212(0, $M^+$); m/e, 170(1), 152(3), 124(5), 110(6), 96(26), 82(34), 68(18), 57(33), 43(100).

Isomère (E)

RMN($^1H$, 360MHz, $CDCl_3$) : 0,88(t, J=7, 3H) ; 1,27(m, 12H) ; 1,37(m, 2H) ; 1,99(q, J=7, 2H) ; 2,11(s, 3H) ; 5,41(dt, $J_1$=7, $J_2$=12, 1H) ; 7,06(d, J=12, 1H) δ ppm.
RMN($^{13}C$, 90MHz, $CDCl_3$) : 14,1(q) ; 20,7(q) ; 22,7(t) ; 27,3(t) ; 29,1-29,6(t) ; 29,1-29,6(t) ; 29,1-29,6(t) ; 29,1-29,6(t) ; 29,1-29,6(t) ; 31,9(t) ; 115,1(d) ; 135,4(d) ; 168,3(s) δ ppm.
SM : 212(0, $M^+$); m/e, 170(1), 152(4), 124(7), 110(8), 96(34), 82(41), 68(19), 57(39), 43(100).

g. acétate de 3-méthyl-5-phényl-1-pentényle

Isomère (Z)

RMN($^1H$, 360MHz, $CDCl_3$) : 1,02(d, J=7, 3H) ; 1,54(m, 1H) ; 1,69(m, 1H) ; 2,10(s, 3H) ; 2,48-2,78(m, 3H) ; 4,72(dd, $J_1$=6, $J_2$=10, 1H) ; 7,03(d, J=6, 1H) ; 7,17(m, 3H) ; 7,26(m, 2H) δ ppm.
RMN($^{13}C$, 90MHz, $CDCl_3$) : 20,7(q) ; 21.0(q) ; 33,7(t) ; 38,9(t) ; 29,2(d) ; 119,8(d) ; 125,6(d) ; 128,2(d) ; 128,2(d) ; 128,4(d) ; 128,4(d) ; 133,4(d) ; 142,5(s) ; 168,1(s) δ ppm.
SM : 218(1, $M^+$); m/e, 176(3), 158(39), 143(27), 131(10), 117(6), 104(16), 91(45), 71(43), 65(13), 51(7), 43(100).

Isomère (E)

RMN($^1H$, 360MHz, $CDCl_3$) : 1,06(d, J=7, 3H) ; 1,62(m, 2H) ; 2,12(s, 3H) ; 2,18(m, 1H) ; 2,60(m, 2H) ; 5,33 (dd, $J_1$=9, $J_2$=13, 1H) ; 7,09(d, J=13, 1H) ; 7,17(m, 3H) ; 7,27(m, 2H) δ ppm.
RMN($^{13}C$, 90MHz, $CDCl_3$) : 20,7(q) ; 21.0(q) ; 33,5(t) ; 38,8(t) ; 32,1(d); 120,3(d) ; 125,7(d) ; 128,3(d) ; 128,3(d) ; 128,4(d) ; 128,4(d) ; 134,9(d) ; 142,3(s) ; 168,3(s) δ ppm.
SM : 218(1, $M^+$); m/e, 176(4), 158(34), 143(25), 131(9), 117(5), 104(16), 91(44), 71(48), 65(12), 51(6), 43(100).

h. octanoate de 3-méthyl-5-phényl-1-pentényle

Isomère (Z)

RMN($^1H$, 360MHz, $CDCl_3$) : 0,89(t, J=7, 3H) ; 1,02(d, J=7, 3H) ; 1,30(m, 8H) ; 1,49-1,76(m, 4H) ; 2,36(t, J=7, 2H) ; 2,48-2,78(m, 3H) ; 4,72(dd, $J_1$=7, $J_2$=10, 1H) ; 7,05(d, J=7, 1H) ; 7,17(m, 3H) ; 7,26(m, 2H) δ ppm.
RMN($^{13}C$, 90MHz, $CDCl_3$) : 14,1(q) ; 21.0(q) ; 22,6(t) ; 24,7(t) ; 28,9(t) ; 29,0(t) ; 31,7(t) ; 33,7(t) ; 34,1(t) ; 38,9(t) ; 29,3(d) ; 119,7(d) ; 125,6(d) ; 128,2(d) ; 128,2(d) ; 128,4(d) ; 128,4(d) ; 133,4(d) ; 142,5(s) ; 171,0 (s) δ ppm.
SM : 308(0, $M^+$); m/e, 176(8), 158(78), 143(18), 127(100), 104(36), 91(51), 71(10), 57(71), 43(14).

Isomère (E)

RMN($^1H$, 360MHz, $CDCl_3$) : 0,89(t, J=7, 3H) ; 1,07(d, J=7, 3H) ; 1,30(m, 8H) ; 1,65(m, 4H) ; 2,18(m, 1H) ; 2,37(t, J=7, 2H) ; 2,60(m, 2H) ; 5,33(dd, $J_1$=8, $J_2$=12, 1H) ; 7,10(d, J=12, 1H) ; 7,17(m, 3H) ; 7,27(m, 2H) δ ppm.
RMN($^{13}C$, 90MHz, $CDCl_3$) : 14,1(q) ; 21.0(q) ; 22,6(t) ; 24,7(t) ; 28,9(t) ; 29,0(t) ; 31,6(t) ; 33,6(t) ; 34,1(t) ; 38,9(t) ; 32,1(d) ; 120,1(d) ; 125,7(d) ; 128,3(d) ; 128,3(d) ; 128,4(d) ; 128,4(d) ; 135,0(d) ; 142,54(s) ; 171,1(s) δ ppm.
SM : 308(0, $M^+$); m/e, 198(1), 176(10), 158(66), 143(20), 127(95), 104(34), 91(56), 71(13), 57(100), 43 (20).

i. 3-acétoxy-2-pentyl-2-cydopentène-1-acétate de méthyle

RMN($^1H$, 360MHz, $CDCl_3$) : 0,88(t, J=7, 3H) ; 1,28(m, 5H) ; 1,41(m, 1H) ; 1,61(m, 1H) ; 1,79(m, 1H) ; 2,14(s, 3H) ; 2,03-2,26(m, 3H) ; 2,46(m, 2H) ; 2,56(dd, $J_1$=4, $J_2$=15, 1H) ; 3,04(m, 1H) ; 3,68(s, 3H) δ ppm.
RMN($^{13}C$, 90MHz, $CDCl_3$) : 14,0(q) ; 20,7(q) ; 51,5(q) ; 22,4(t) ; 24,5(t) ; 26,8(t); 27,2(t) ; 29,6(t) ; 31,7(t) ; 38,6 (t) ; 39,7(d) ; 128,3(s) ; 145,4(s) ; 168,5(s) ; 173,2(s) δ ppm.
SM : 268(1, $M^+$); m/e, 226(10), 208(2), 195(3), 169(3), 153(100), 137(4), 123(3), 109(10), 97(35), 83(10), 67 (9), 55(8), 43(31).

j. 3-octanoyloxy-2-pentyl-1-cyclopentène-1-acétate de méthyle

RMN($^1$H, 360MHz, $CDCl_3$): 0,88(t, J=7, 3H); 0,89(t, J=7, 3H) ; 1,30(m, 14H) ; 1,53-1,72(m, 3H) ; 1,79(m, 1H) ; 2,08(m, 1H) ; 2,19(m, 2H) ; 2,31-2,51(m, 4H) ; 2,57(dd, $J_1$=4, $J_2$=15, 1H) ; 3,05(m, 1H) ; 3,68(s, 3H) δ ppm.
RMN($^{13}$C, 90MHz, $CDCl_3$) : 14,0(q) ; 14,1(q) ; 51,5(q) ; 22,4(t) ; 22,6(t) ; 24,5(t) ; 25,0(t) ; 26,8(t) ; 27,1(t) ; 28,9 (t) ; 29,1(t) ; 29,6(t) ; 31,7(t) ; 31,7(t) ; 34,2(t) ; 38,6(t) ; 39,6(d) ; 128,2(s); 145,3(s); 171,5(s); 173,3(s) δ ppm.
SM : 352(0, $M^+$); m/e, 279(2), 226(92), 208(3), 194(4), 169(4), 153(100), 127(12), 109(7), 97(6), 81(5), 67(5), 57(22), 43(10).

Exemple 4

Essai sur textiles

Plusieurs tests ont été exécutés sur des textiles, dans des conditions variées, ces textiles ayant été traités selon la méthode générale suivante.

**Méthode générale de traitement des textiles**

Une serviette éponge standard de 32 g a été placée dans un récipient Linitest ® (origine: Hanau, Allemagne) contenant 1,3 g d'une base standard de détergent en poudre comprenant 1% en poids de lipase (Lipolase ® 100T ; origine : Novo Nordisk, Danemark) et 260 ml d'eau. La serviette a été lavée pendant 30 min à 40°. Elle a ensuite été sortie du récipient et rincée à l'aide de 3x200 ml d'eau. Par la suite on l'a immergée pendant 5 min dans 200 ml d'eau contenant 0,6 g d'une base d'adoucissant textile qui comprenait un certain pourcentage en poids, compris entre 0,05 et 1%, de composé (I) selon l'invention ou, le cas échéant, de l'alcool, aldéhyde ou cétone correspondants. La serviette a alors été essorée sans avoir été rincée et séchée sur une corde à linge.
Cette méthode équivaut à un lavage dans une vraie machine à laver de 5 kg de linge dans environ 20 l d'eau, avec un rinçage de 4x201 d'eau, l'adoucissant étant appliqué dans la dernière eau de rinçage.
La base adoucissante textile utilisée avait la composition suivante :

| Ingrédients | % en poids |
|---|---|
| Arquad 2 HT 1) (75%) | 5,00 |
| Formalin (40%) | 0,20 |
| Eau déminéralisée | 94,69 |
| Colorant 2) | 0,11 |
| Total | 100,00 |

1) origine : Akzo, Hollande
2) Colanyl Blau AR/sol. à 10% ; origine : Hoechst, Allemagne

Dans deux essais séparés, des serviettes éponge ont été traitées selon cette méthode générale, en utilisant en tant qu'additif de l'adoucissant textile respectivement de l'hexadécanoate de déc-9-ényle (1% en poids) dans l'essai A et déc-9-én-1-ol (1% en poids) dans l'essai B.
Les deux serviettes ont été soumises pour évaluation à un test à l'aveugle 24 h après avoir été sorties de l'appareil Linitest ® . Le panel d'évaluation était constitué par huit personnes, auxquelles on avait fourni au départ une mouillette qui avait été trempée dans du déc-9-én-1-ol afin de les familiariser avec l'odeur de ce composé. Le panel devait par la suite sentir les deux serviettes susmentionnées et indiquer s'ils pouvaient identifier dans l'une ou l'autre des serviettes l'odeur du déc-9-én-1-ol. Cinq des huit membres du panel ont reconnu l'odeur de cet alcool dans la serviette traitée dans l'essai A et indiqué qu'elle était très puissante, alors que seul un de ceux-ci a reconnu la même odeur sur la serviette traitée dans l'essai B.
Une évaluation supplémentaire des deux serviettes effectuée par le même panel, dans des conditions identiques, 24 h plus tard, c'est-à-dire 48 h après que les serviettes aient été sorties de l'appareil Linitest ® , a révélé qu'aucun des membres du panel ne pouvait identifier l'odeur du déc-9-én-1-ol sur la serviette de l'essai B, alors que six d'entre eux l'ont identifiée sans hésitation sur la serviette sortie de l'essai A.
Des résultats similaires ont été obtenus lorsque les deux composés susmentionnés étaient présents dans l'adoucissant textile à raison de 0,5% en poids.

Exemple 5

Essai sur textiles

Neuf serviettes éponge standard (32 g chacune), numérotées de 1 à 9, ont été traitées séparément et de façon identique comme il est décrit dans l'Exemple 4, le seul élément changeant étant l'additif incorporé dans l'adoucissant textile (à 1% en poids) et décrit dans le Tableau 1 ci-après.

TABLEAU 1

| SERVIETTE | ADDITIF DE L'ADOUCISSANT |
|---|---|
| 1 | déc-9-én-1-ol |
| 2 | octanoate de déc-9-ényle |
| 3 | nonanoate de déc-9-ényle |
| 4 | décanoate de déc-9-ényle |
| 5 | undécanoate de déc-9-ényle |
| 6 | dodécanoate de déc-9-ényle |
| 7 | tridécanoate de déc-9-ényle |
| 8 | tétradécanoate de déc-9-ényle |
| 9 | pentadécanoate de déc-9-ényle |

24 H après avoir été enlevées des machines Linitest ® , les neuf serviettes éponge ont été soumises pour évaluation à l'aveugle à un panel constitué de dix personnes, lesquelles, après avoir senti le déc-9-én-1-ol sur une mouillette, devaient indiquer si elles reconnaissaient l'odeur de cet alcool sur une ou plusieurs serviettes. Le résultat de cette évaluation est résumé dans le Tableau 2 ci-après (seules les identifications catégoriques ont été prises en considération) :

TABLEAU 2

| SERVIETTE | Nombre de membres du panel ayant reconnu l'odeur |
|---|---|
| 1 | 0 |
| 2 | 10 |
| 3 | 8 |
| 4 | 3 |
| 5 | 6 |
| 6 | 10 |
| 7 | 8 |
| 8 | 7 |
| 9 | 9 |

Dans un test similaire effectué 48 h après avoir enlevé les serviettes des machines Linitest ® , les membres du panel étaient toujours unanimes en ce qui concernait la serviette 1, c'est-à-dire qu'ils ne pouvaient pas sentir l'odeur du déc-9-én-1-ol sur cette serviette, alors qu'une majorité d'entre eux la jugeait toujours très intense sur toutes les autres serviettes.

Exemple 6

Essai sur textiles

Six serviettes éponge standard (32 g chacune), numérotées de 1 à 6, ont été traitées séparément et de façon identique comme il est décrit dans l'Exemple 4, le seul élément changeant étant l'additif incorporé (à 1% en poids) dans l'adoucissant textile et décrit dans le Tableau 3 ci-après :

TABLEAU 3

| SERVIETTE | ADDITIF DE L'ADOUCISSANT |
|---|---|
| 1 | déc-9-én-1-ol |
| 2 | oxalate de bis(déc-9-ényl) |
| 3 | malonate de bis(déc-9-ényl) |
| 4 | butanedioate de bis(déc-9-ényl) |
| 5 | pentanedioate de bis(déc-9-ényl) |
| 6 | hexanedioate de bis(déc-9-ényl) |

24 H après avoir été enlevées des machines Linitest ® , les six serviettes éponge ont été soumises pour évaluation à l'aveugle à un panel constitué de dix personnes, lesquelles, après avoir senti le déc-9-én-1-ol sur une mouillette, devaient indiquer si elles reconnaissaient l'odeur de cet alcool sur une ou plusieurs serviettes. Le résultat de cette évaluation est résumé dans le Tableau 4 ci-après (seules les identifications catégoriques ont été prises en considération) :

TABLEAU 4

| SERVIETTE | Nombre de membres du panel ayant reconnu l'odeur après 24 h |
|---|---|
| 1 | 0 |
| 2 | 10 |
| 3 | 6 |
| 4 | 8 |
| 5 | 9 |
| 6 | 7 |

Le même test, effectué 48 h après avoir sorti les serviettes des machines Linitest ® , a produit les résultats résumés dans le Tableau 5 ci-après:

TABLEAU 5

| SERVIETTE | Nombre de membres du panel ayant reconnu l'odeur après 48 h |
|---|---|
| 1 | 0 |
| 2 | 10 |
| 3 | 7 |
| 4 | 6 |
| 5 | 8 |
| 6 | 7 |

Exemple 7

Essai sur textiles

Six serviettes éponge standard (32 g chacune), numérotées de 1 à 6, ont été traitées séparément et de façon identique comme il est décrit dans l'Exemple 4. Le contenu en lipase du détergent était maintenant de 3% en poids et la quantité d'adoucissant ajouté à la dernière eau de rinçage était maintenant de 1,2 g. L'additif incorporé (à 1% en poids) dans l'adoucissant textile dans chaque cas est inscrit au Tableau 6 suivant :

TABLEAU 6

| SERVIETTE | ADDITIF DE L'ADOUCISSANT |
|---|---|
| 1 | (E)-3,7-diméthyl-octa-2/6-dién-1-ol |
| 2 | oxalate de (E,E)-bis(3,7-diméthyl-octa-2,6-diényl) |
| 3 | malonate de (E,E)-bis(3,7-diméthyl-octa-2,6-diényl) |
| 4 | butanedioate de (E,E)-bis(3,7-diméthyl-octa-2,6-diényl) |

TABLEAU 6 (suite)

| SERVIETTE | ADDITIF DE L'ADOUCISSANT |
|---|---|
| 5 | pentanedioate de (E,E)-bis(3,7-diméthyl-octa-2,6-diényl) |
| 6 | hexanedioate de (E,E)-bis(3,7-diméthyl-octa-2,6-diényl) |

24 H après avoir été enlevées des machines Linitest® , les six serviettes éponge ont été soumises pour évaluation à l'aveugle à un panel constitué de dix personnes, lesquelles, après avoir senti le (E)-3,7-diméthyl-octa-2,6-dién-1-ol sur une mouillette, devaient indiquer si elles reconnaissaient l'odeur de cet alcool sur une ou plusieurs serviettes. Le résultat de cette évaluation est résumé dans le Tableau 7 ci-après (seules les identifications catégoriques ont été prises en considération) :

TABLEAU 7

| SERVIETTE | Nombre de membres du panel ayant reconnu l'odeur |
|---|---|
| 1 | 2 |
| 2 | 7 |
| 3 | 7 |
| 4 | 9 |
| 5 | 7 |
| 6 | 8 |

Exemple 8

Essai sur textiles

Deux serviettes éponge standard (32 g chacune), numérotées 1 et 2, ont été traitées séparément et de façon identique comme il est décrit dans l'Exemple 4, le seul élément changeant étant l'additif incorporé dans l'adoucissant textile et décrit dans le Tableau 8 ci-après:

TABLEAU 8

| SERVIETTE | ADDITIF DE L'ADOUCISSANT |
|---|---|
| 1 | 3,7-diméthyl-oct-6-én-l-ol |
| 2 | hexadécanoate de 3,7-diméthyl-oct-6-ényle |

24 H après avoir été enlevées des machines Linitest ® , les deux serviettes éponge ont été soumises pour évaluation à l'aveugle à un panel constitué de onze personnes, lesquelles, après avoir senti le (E)-3,7-diméthyl-oct-6-én-l-ol sur une mouillette, devaient indiquer si elles reconnaissaient l'odeur de cet alcool sur l'une ou l'autre des serviettes. Le résultat de cette évaluation est résumé dans le Tableau 9 ci-après (seules les identifications catégoriques ont été prises en considération) :

TABLEAU 9

| SERVIETTE | Nombre de membres du panel ayant reconnu l'odeur |
|---|---|
| 1 | 0 |
| 2 | 7 |

Un test similaire a été effectué avec deux serviettes traitées avec la même base adoucissante mais contenant seulement 0,5% en poids des composés répertoriés dans le Tableau 8. L'évaluation du panel a montré qu'aucun des membres du panel ne pouvait identifier l'odeur de l'alcool sur la serviette 1, alors que 7 des 11 membres du panel étaient sûrs de la sentir sur la serviette 2. Des résultats analogues ont encore été obtenus avec un dosage de 0,1% en poids des composés susmentionnés.

Example 9

Essai sur textiles

Trois serviettes éponge standard (32 g chacune), numérotées de 1 à 3, ont été traitées séparément et de façon identique comme il est décrit dans l'Exemple 4, le seul élément changeant étant l'additif incorporé (à 1% en poids) dans l'adoucissant textile et décrit dans le Tableau 10 ci-après :

TABLEAU 10

| SERVIETTE | ADDITIF DE L'ADOUCISSANT |
|---|---|
| 1 | 3-méthyl-5-phénylpentan-1-ol |
| 2 | octanoate de 3-méthyl-5-phénylpentyle |
| 3 | hexadécanoate de 3-méthyl-5-phénylpentyle |

24 H après avoir été enlevées des machines Linitest ® , les trois serviettes éponge ont été soumises pour évaluation à l'aveugle à un panel constitué de dix personnes, lesquelles, après avoir senti le 3-méthyl-5-phénylpentan-1-ol sur une mouilllette, devaient indiquer si elles reconnaissaient l'odeur de cet alcool sur une ou plusieurs serviettes. Le résultat de cette évaluation est résumé dans le Tableau 11 ci-après (seules les identifications catégoriques ont été prises en considération) :

TABLEAU 11

| SERVIETTE | Nombre de membres du panel ayant reconnu l'odeur |
|---|---|
| 1 | 2 |
| 2 | 6 |
| 3 | 5 |

Exemple 10

Essai sur textiles

Trois serviettes éponge standard (32 g chacune) ont été traitées séparément en suivant la méthode générale décrite dans l'Exemple 4, deux de ces serviettes (1 et 2) en utilisant du déc-9-én-1-ol en tant qu'additif de l'adoucissant, et la serviette 3 en utilisant comme additif de l'hexadécanoate de déc-9-ényle, ces additifs ayant été ajoutés à l'adoucissant textile dans une concentration de 0,5% en poids.
Les trois serviettes ont été évaluées à l'aveugle par un panel de 12 personnes 24 h après avoir été sorties des machines Linitest ® . On a demandé aux membres du panel s'ils pouvaient discerner une différence quelconque entre les serviettes et, le cas échéant, s'ils pouvaient qualifier cette différence. Le panel avait senti auparavant des mouillettes trempées dans du déc-9-én-1-ol, et était conscient que l'on attendait une évaluation des trois serviettes eu égard l'odeur particulière de ce composé.
En résultat de l'évaluation, 8 des 12 membres du panel ont choisi la serviette 3 comme étant celle qui était olfactivement distincte et ont indiqué qu'elle développait l'odeur caractéristique du déc-9-én-1-ol, alors que les deux autres n'exhalaient aucune odeur distinctive.
Le même test a été effectué avec 1% d'additif dans l'adoucissant, au lieu de 0,5%. Dans ce cas, 9 des 12 membres du panel ont de nouveau choisi la serviette 3 comme étant celle qui développait l'odeur de l'alcool susmentionné et ont jugé que cette odeur était encore plus puissante que dans le test précédent. D'autre part, le panel a trouvé que les deux autres serviettes n'avaient toujours pas de fragrance distinctive.

Exemple 11

Essai sur textiles

Deux serviettes éponge standard (32 g chacune), numérotées 1 et 2, ont été traitées séparément et de façon identique comme il est décrit dans l'Exemple 4, le seul élément changeant étant l'additif incorporé dans l'adoucissant textile, à raison de 0,05% en poids, et décrit dans le Tableau 12 ci-après:

TABLEAU 12

| SERVIETTE | ADDITIF DE L'ADOUCISSANT |
|---|---|
| 1 | 3,7-diméthyl-2,6-octadién-1-ol |
| 2 | hexadécanoate de 3,7-diméthyl-2,6-octadiényle |

24 H après avoir été enlevées des machines Linitest ® , les deux serviettes éponge ont été soumises pour évaluation à l'aveugle à un panel constitué de douze personnes, lesquelles, après avoir senti le 3,7-diméthyl-2,6-octadién-1-ol sur une mouillette, devaient indiquer si elles reconnaissaient l'odeur de cet alcool sur l'une ou l'autre des serviettes. Le résultat de cette évaluation est résumé dans le Tableau 13 ci-après (seules les identifications catégoriques ont été prises en considération) :

TABLEAU 13

| SERVIETTE | Nombre de membres du panel ayant reconnu l'odeur |
|---|---|
| 1 | 0 |
| 2 | 8 |

Exemple 12

Essai sur textiles

Deux serviettes éponge standard (32 g chacune), numérotées 1 et 2, ont été traitées séparément et de façon identique comme il est décrit dans l'Exemple 4, mais en ajoutant à l'adoucissant textile 1% en poids des additifs cités dans le Tableau 14 ci-après :

TABLEAU 14

| SERVIETTE | ADDITIF DE L'ADOUCISSANT |
|---|---|
| 1 | 3,7-diméthyl-6-octénal |
| 2 | octanoate de 3,7-diméthyl-1,6-octadiényle |

24 H après avoir été enlevées des machines Linitest® , les deux serviettes éponge ont été soumises pour évaluation à l'aveugle à un panel constitué de onze personnes, lesquelles, après avoir senti le 3,7-diméthyl-6-octénal sur une mouillette, devaient indiquer si elles reconnaissaient l'odeur de cet aldéhyde sur l'une ou l'autre des serviettes. Le résultat de cette évaluation est résumé dans le Tableau 15 ci-après (seules les identifications catégoriques ont été prises en considération) :

TABLEAU 15

| SERVIETTE | Nombre de membres du panel ayant reconnu l'odeur |
|---|---|
| 1 | 4 |
| 2 | 11 |

Exemple 13

Essai sur textiles

Deux serviettes éponge standard (32 g chacune), numérotées 1 et 2, ont été traitées séparément et de façon identique comme il est décrit dans l'Exemple 4, le seul élément changeant étant l'additif incorporé (à 1% en poids) dans l'adoucissant textile et décrit dans le Tableau 16 ci-après :

TABLEAU 16

| SERVIETTE | ADDITIF DE L'ADOUCISSANT |
|---|---|
| 1 | 3-méthyl-5-phényl-1-pentanal |
| 2 | octanoate de 3-méthyl-5-phényl-1-pentényle |

24 H après avoir été enlevées des machines Linitest ® , les deux serviettes éponge ont été soumises pour évaluation à l'aveugle à un panel constitué de douze personnes, lesquelles, après avoir senti le 3-méthyl-5-phényl-1-pentanal sur une mouillette, devaient indiquer si elles reconnaissaient l'odeur de cet aldéhyde sur l'une ou l'autre des serviettes. Le résultat de cette évaluation est résumé dans le Tableau 17 ci-après (seules les identifications catégoriques ont été prises en considération) :

TABLEAU 17

| SERVIETTE | Nombre de membres du panel ayant reconnu l'odeur |
|---|---|
| 1 | 2 |
| 2 | 8 |

Exemple 14

Essai sur textiles

Trois serviettes éponge standard (32 g chacune) ont été traitées séparément en suivant la méthode générale décrite dans l'Exemple 4, deux de ces serviettes (1 et 2) en utilisant du 2-méthyl-1-undécanal en tant qu'additif de l'adoucissant, et la serviette 3 en utilisant comme additif de l'octanoate de 2-méthyl-1-undécényle, ces additifs ayant été ajoutés à l'adoucissant textile dans une concentration de 0,1% en poids.

Les trois serviettes ont été évaluées à l'aveugle par un panel de 12 personnes 24 h après avoir été sorties des machines Linitest ® . On a demandé aux membres du panel s'ils pouvaient discerner une différence quelconque entre les serviettes et, le cas échéant, s'ils pouvaient qualifier cette différence. Le panel avait senti auparavant des mouillettes trempées dans du 2-méthyl-1-undécanal, et était conscient que l'on attendait une évaluation des trois serviettes eu égard l'odeur particulière de ce composé.
En résultat de l'évaluation, 10 des 12 membres du panel ont choisi la serviette 3 comme étant celle qui était olfactivement distincte et ont indiqué qu'elle développait l'odeur caractéristique du 2-méthyl-1-undécanal, alors que les deux autres n'exhalaient aucune odeur distinctive.

Exemple 15

Essai sur textiles traités dans une machine à laver

Deux lots standard de textiles en coton, comprenant chacun 30 serviettes éponge (32 g chacune) ont été placés dans deux machines à laver séparées, chacune contenant 130 g d'une base standard de détergent en poudre comprenant 1% en poids de lipase (Lipolase ® 100T ; origine : Novo Nordisk, Danemark) placée dans le compartiment à détergent. Dans le récipient destiné à l'adoucissant, on a placé dans l'un des cas 100 g d'une base adoucissante telle que décrite à l'Exemple 4 parfumée avec 0,1% en poids de 2-méthyl-1-undécanal, alors que l'adoucissant (100 g) placé dans la deuxième machine contenait 0,1% en poids d'octanoate de 2-méthyl-1-undécényle. Les lots de serviettes ont ensuite été lavés avec un cycle de lavage normal, à 40°, sans prélavage, et séchés à l'air.
Les lots de serviettes ont été évalués à l'aveugle 24 h après leur sortie de la machine par un panel de 36 personnes. Ces dernières devaient indiquer si elles trouvaient une distinction quelconque dans l'odeur de trois lots de serviettes, parmi lesquels deux étaient identiques et avaient été traités avec l'adoucissant contenant le 2-méthyl-1-undécanal (lots 1 et 2) et le troisième avait été traité avec l'octanoate de 2-méthyl-1-undécényle (lot 3), sans qualifier la nature du caractère distinctif. Par ailleurs, on leur demandait également d'indiquer si l'un des lots possédait une odeur plus intense.
Des 36 panélistes, 24 ont identifié le lot de textiles 3 comme étant celui qui différait des deux autres, d'un point de vue olfactif, et indiqué que ce lot développait une odeur plus intense que les deux autres.

Un test analogue a été exécuté mais avec deux lots de textiles uniquement, le lot A ayant été traité avec un adoucissant qui contenait 0,05% en poids de 2-méthyl-1-undécanal et le lot B avec un adoucissant contenant 0,05% en poids de l'énol-ester correspondant, à savoir le 2-méthyl-1-undécényle.
Le panel de 36 personnes qui a évalué l'odeur des deux lots de textiles A et B, sans connaître la nature des ingrédients de parfumage, devait indiquer lequel des lots exhalait une odeur plus intense et lequel était préféré du point de vue de la perception de fraîcheur et propreté du linge.
De l'avis catégorique de 32 parmi les 36 panélistes, le lot de textiles B était beaucoup plus "parfumé" que le lot A et était perçu comme plus propre, avec une odeur plus fraîche.

Exemple 16

Essais sur textiles traités dans une machine à laver

On a préparé une composition parfumante de base en mélangeant les ingrédients suivants :

| Ingrédients | % en poids |
|---|---|
| Acétate de benzyle | 30 |
| Acétate de 1,1-diméthyl-2-phényléthyle | 25 |
| 10-Undécénal | 3 |
| Aldéhyde hexylcinnamique | 150 |
| Anthranilate de méthyle dist. | 10 |
| Cedroxyde ® 1) | 40 |
| Ambrox ® 2) DL à 10% * | 2 |
| Acétate de verdyle 3) | 30 |
| α-Damascone à 10% * | 3 |
| Dihydromyrcenol 4) | 80 |
| Dynascone ® 5) à 10% * | 2 |
| Eugénol | 7 |
| Exaltex ® 6) | 50 |
| Galbex ® 7) 183 | 8 |
| Geranyl nitrile | 1 |
| Indol à 10% ** | 5 |
| Iralia ® 8) | 20 |
| Isoeugénol | 3 |
| Lilial ® 9) | 50 |
| Linalol | 30 |
| Lorysia ® 10) | 50 |
| Mayol ® 11) | 10 |
| Hédione ® 12) | 50 |
| Oxyde de rose 13) à 10% * | 5 |
| Phénéthylol | 60 |
| Polysantol ® 14) | 3 |
| Essence d'orange | 40 |
| Salicylate de benzyle | 30 |
| Scentenal ® 15) | 3 |
| Terpinéol | 25 |

* dans le dipropylneglycol (DIPG)
** dans la trithanolamine
1) triméthyl-13-oxabicydo[10.1.0]tridéca-4,8-dine ; origine : Firmenich SA, Genève, Suisse
2) dodécahydro-3,6,6,9a-tétraméthyl-naphto[2,1-b]furane ; origine : Firmenich SA, Genève, Suisse
3) acétate de tricyclo[5.2.1.$0^{2,6}$ ]déc-3-én-8-yle ; origine : Givaudan-Roure, Vernier, Suisse
4) 2,6-diméthyl-7-océtn-2-ol ; origine : International Flavors & Fragrances, USA
5) 1-(5,5-diméthyl-1-cyclohéx-1-yl)-4-pentén-1-one ; origine : Firmenich SA, Genève, Suisse
6) oxacydohexadécan-2-one ; origine : Firmenich SA, Genève, Suisse
7) origine : Firmenich SA, Genève, Suisse
8) méthylionone ; origine : Firmenich SA, Genève, Suisse
9) 3-(4-tert-butyl-1-phényl)-2-méthylpropanal ; origine : Givaudan-Roure, Vernier, Suisse
10) acétate de 4-(1,1-diméthyléthyl)-cyclohexanol ; origine : Firmenich SA, Genève, Suisse
11) cis-4-(1-méthyléthyl)-cyclohexaneméthanol ; origine: Firmenich SA, Genève, Suisse
12) ester méthylique de l'acide 3-oxo-1-pentyl-cyclopentaneacétique ; origine : Firmenich SA, Genève, Suisse
13) origine : Firmenich SA, Genève, Suisse
14) 3,3-diméthyl-5-(2,2,3-triméthyl-3-cyclopentén-1-yl)-4-pentén-2-ol ; origine : Firmenich SA, Genève, Suisse
15) octahydro-5-méthoxy-4,7-méthano-1H-indène-2-carboxaldéhyde ; origine : Firmenich SA, Genève, Suisse

(suite)

| Ingrédients | % en poids |
|---|---|
| Veloutone [16)] | 2 |
| Verdox ® [17)] | 20 |
| β-Naphtol méthyl-éther | 2 |
| Zestover ® [18)] | 1 |
| **Total** | 850 |

16) 2,2,5-triméthyl-5-pentyl-cyclopentanone ; origine : Firmenich SA, Genève, Suisse
17) acétate de 2-tert-butyl-cyclohexanyle ; origine : International Flavors & Fragrances, USA
18) 2,4-diméthyl-3-cyclohexène-1-carbaldéhyde ; origine: Firmenich SA, Genève, Suisse

Avec cette composition de base, on a préparé deux compositions parfumantes par addition de 150 parties en poids de géraniol ou 3,7-diméthyl-octa-2,6-diénol (composition A), respectivement 150 parties en poids d'hexadécanoate de 3,7-diméthyl-octa-2,6-diényle (composition B).
Ces deux compositions parfumantes ont ensuite été ajoutées, à raison de 0,6% en poids, à une base d'adoucissant textile non parfumée pour préparer deux échantillons, A et respectivement B, d'adoucissant parfumé.
Dans deux machines à laver le linge identiques, on a traité deux lots standard de textiles, comportant chacun 1400 g de linge varié, comprenant des tissus éponge, des textiles en coton et des textiles en mélange coton/polyester. Chaque machine a été chargée avec 130 g d'une base détergente standard non parfumée contenant 1% de lipase (Lipolase ® 100T). L'une des machines a également été chargée avec 100 g de l'échantillon A d'adoucissant, comportant le géraniol, alors que dans la deuxième machine on a ajouté 100 g de l'échantillon d'adoucissant B.
Le linge a ensuite été lavé à 40° dans un cycle de lavage normal, sans prélavage, et séché à l'air pendant 24 h.
Trois lots de textiles, dont deux étaient identiques et avaient été traités avec l'échantillon A et le troisième avec l'échantillon B, ont ensuite été évalués à l'aveugle par un panel de 20 personnes, qui devaient identifier le lot de textiles possédant une odeur distincte des deux autres, sans qualifier la nature du caractère distinctif. Les panélistes devaient également indiquer lequel des lots de textiles était le plus parfumé.
En résultat de ce test, 14 panélistes percevaient une distinction dans l'odeur du lot de textiles traité avec l'échantillon B de l'adoucissant textile, alors que 10 parmi ces 14 ont indiqué que ce même lot était le plus parfumé.
Lorsqu'on a répété l'évaluation après 48 h de séchage à l'air, on a obtenu des résultats analogues.

## Revendications

1. Procédé pour le parfumage de textiles soumis au lavage en présence d'un détergent contenant une lipase, suivi facultativement d'un traitement avec un adoucissant textile, le procédé étant **caractérisé en ce que** ledit détergent et/ou ledit adoucissant contiennent un composé de formule

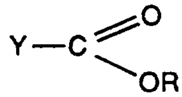

(I)

dans laquelle

a. R représente un radical dérivé d'un alcool odorant de formule ROH et Y représente un radical alkyle de $C_7$ à $C_{24}$, linéaire ou ramifié, saturé ou insaturé, ou un groupe $-(CH_2)_nCOOR$ dans lequel R est défini comme ci-dessus et n est un nombre entier de 0 à 6 ;
ou
b. Y représente un radical alkyle de $C_7$ à $C_{24}$, linéaire ou ramifié, saturé ou insaturé, et R représente un groupe de formule

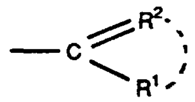

dans laquelle, soit $R^1$ représente l'hydrogène et $R^2$ représente un radical alkylidène dérivé d'un aldéhyde odorant de formule

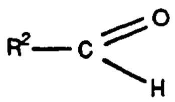

soit $R^2$ représente un radical alkylidène et $R^1$ un radical alkyle, $R^1$ et $R^2$ étant dérivés d'une cétone odorante de formule

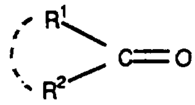

et pouvant faire partie d'un cycle tel qu'indiqué par la ligne pointillée, contenant de 5 à 18 atomes de carbone, éventuellement substitué.

**2.** Procédé pour le parfumage de textiles soumis au lavage en présence d'un détergent contenant une lipase, lequel procédé comprend le traitement desdits textiles, après le cycle de lavage, avec un adoucissant textile contenant un composé de formule (I) telle que définie à la revendication 1.

**3.** Procédé pour le lavage de textiles, comprenant un cycle de lavage en présence d'un détergent contenant une lipase, suivi facultativement d'un traitement avec un adoucissant textile, le procédé étant **caractérisé en ce que** ledit détergent et/ou ledit adoucissant textile contiennent un composé de formule

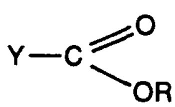

(I)

telle que définie à la revendication 1.

**4.** Procédé pour le lavage de textiles, comprenant un cycle de lavage en présence d'un détergent contenant une lipase, suivi du traitement desdits textiles avec un adoucissant textile contenant un composé de formule (I) telle que définie à la revendication 1.

**5.** Procédé pour prolonger l'effet de - diffusion de l'odeur caractéristique d'un alcool odorant de formule ROH développée par des textiles, **caractérisé en ce qu'**on soumet ces textiles à un cycle de lavage en présence d'un détergent contenant une lipase et, facultativement, à un traitement subséquent avec un adoucissant textile, ledit détergent et/ou ledit adoucissant textile contenant un composé de formule

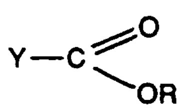

(I)

dans laquelle R représente un radical monovalent dérivé dudit alcool odorant, et Y représente un radical alkyle de C7 à $C_{24}$, linéaire ou ramifié, saturé ou insaturé, ou un groupe $-(CH_2)_nCOOR$ dans lequel R est défini comme ci-dessus et n est un nombre entier de 0 à 6.

**6.** Procédé pour prolonger l'effet de diffusion de l'odeur caractéristique d'un alcool odorant de formule ROH développée par des textiles soumis à lavage avec un détergent contenant une lipase, **caractérisé en ce que** lesdits textiles, après le cycle de lavage, sont traités avec un adoucissant textile contenant un composé de formule

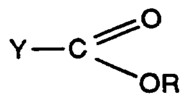 (I)

dans laquelle R représente un radical monovalent dérivé dudit alcool odorant, et Y représente un radical alkyle de $C_7$ à $C_{24}$, linéaire ou ramifié, saturé ou insaturé, ou un groupe $-(CH_2)_nCOOR$ dans lequel R est défini comme ci-dessus et n est un nombre entier de 0 à 6.

**7.** Procédé pour prolonger l'effet de diffusion de l'odeur caractéristique d'un aldéhyde odorant de formule

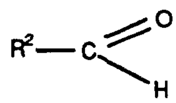

ou d'une cétone odorante de formule

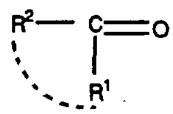

développée par des textiles, **caractérisé en ce qu'**on soumet ces textiles à un cycle de lavage en présence d'un détergent contenant une lipase et, facultativement, à un traitement subséquent avec un adoucissant textile, ledit détergent et ou/ledit adoucissant textile contenant un composé de formule

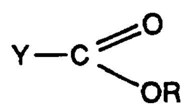 (I)

dans laquelle Y représente un radical alkyle de $C_7$ à $C_{24}$, linéaire ou ramifié, saturé ou insaturé, et R représente un groupe de formule

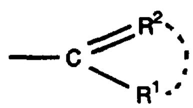

dans laquelle, soit $R^1$ représente l'hydrogène et $R^2$ représente un radical alkylidène dérivé dudit aldéhyde odorant, soit $R^2$ représente un radical alkylidène et $R^1$ un radical alkyle, $R^1$ et $R^2$ étant dérivés de ladite cétone odorante et pouvant faire partie d'un cycle tel qu'indiqué par la ligne pointillée, contenant de 5 à 18 atomes de carbone, éventuellement substitué.

**8.** Procédé pour prolonger l'effet de diffusion de l'odeur caractéristique d'un aldéhyde odorant de formule

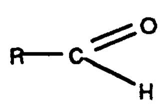

ou d'une cétone odorante de formule

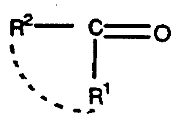

développée par des textiles soumis au lavage avec un détergent contenant une lipase, **caractérisé en ce que** lesdits textiles, après le cycle de lavage, sont traités avec un adoucissant textile contenant un composé de formule

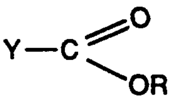 (I)

dans laquelle Y représente un radical alkyle de $C_7$ à $C_{24}$, linéaire ou ramifié, saturé ou insaturé, et R représente un groupe de formule

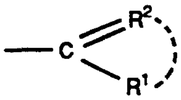

dans laquelle, soit $R^1$ représente l'hydrogène et $R^2$ représente un radical alkylidène dérivé dudit aldéhyde odorant, soit $R^2$ représente un radical alkylidène et $R^1$ un radical alkyle, $R^1$ et $R^2$ étant dérivés de ladite cétone odorante et pouvant faire partie d'un cycle tel qu'indiqué par la ligne pointillée, contenant de 5 à 18 atomes de carbone, éventuellement substitué.

**9.** Procédé selon l'une des revendications 1 à 6 comprenant l'addition d'un composé de formule (I) dans laquelle R est le radical déc-9-én-1-yle, 3,7-diméthyl-octa-2,6-dién-1-yle, 3,7-diméthyl-oct-6-én-1-yle ou 3-méthyl-5-phénylpentyle.

**10.** Procédé selon l'une des revendications 1 à 4, 7 ou 8, comprenant l'addition d'un composé de formule (I) dans laquelle R est le radical 3,7-diméthyl-1,6-octadiényle, 3-méthyl-5-phényl-1-pentényle ou 2-méthyl-1-undécényle.

**11.** Procédé selon l'une des revendications 1 à 10 comprenant l'addition d'un composé de formule (I) dans laquelle Y est un radical alkyle linéaire de $C_{12}$ à $C_{24}$.

**12.** Détergent ou adoucissant textile comprenant un composé de formule (I) telle que définie à la revendication 1.

**13.** Composé de formule

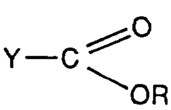 (I)

dans laquelle

a. R représente un radical dérivé d'un alcool odorant de formule ROH et Y représente un radical alkyle de $C_7$ à $C_{24}$, linéaire ou ramifié, saturé ou insaturé, ou un groupe $-(CH_2)_nCOOR$ dans lequel R est défini comme ci-dessus et n est un nombre entier de 0 à 6 ;
ou
b. Y représente un radical alkyle de $C_7$ à $C_{24}$, linéaire ou ramifié, saturé ou insaturé, et R représente un groupe de formule

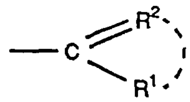

dans laquelle, soit $R^1$ représente l'hydrogène et $R^2$ représente un radical alkylidène dérivé d'un aldéhyde odorant de formule

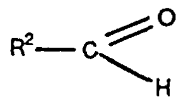

soit $R^2$ représente un radical alkylidène et $R^1$ un radical alkyle, $R^1$ et $R^2$ étant dérivés d'une cétone odorante de formule

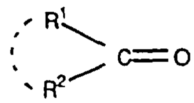

et pouvant faire partie d'un cycle tel qu'indiqué par la ligne pointillée, contenant de 5 à 18 atomes de carbone, éventuellement substitué, étant entendu que l'octanoate de 2-phényléthyle, l'adipate de n-hexyle, l'adipate de cyclohexyle et l'adipate de n-hexylcyclohexyle sont exclus.

**14.** Composé selon la revendication 13, dans lequel R est le radical déc-9-én-1-yle, 3,7-diméthyl-octa-2,6-dién-1-yle, 3,7-diméthyl-oct-6-én-1-yle, 3-méthyl-5-phénylpentyle, 3,7-diméthyl-1,6-octadiényle, 3-méthyl-5-phényl-1-pentényle ou 2-méthyl-1-undécényle.

**15.** Composé selon la revendication 13 ou 14, dans lequel Y est un radical alkyle linéaire de $C_{12}$ à $C_{24}$.

**16.** Composition parfumante contenant au moins un ingrédient parfumant et un solvant ou adjuvant d'usage courant en parfumerie, **caractérisée en ce qu'**elle comprend un composé de formule (I) telle que définie à la revendication 1.

## Patentansprüche

**1.** Verfahren zum Beduften von Textilien, die einer Wäsche in Gegenwart eines eine Lipase enthaltenden Detergens unterzogen werden, gegebenenfalls gefolgt von einer Behandlung mit einem Textilweichspüler, wobei das Verfahren **dadurch gekennzeichnet ist, daß** das Detergens und/oder der Weichspüler eine Verbindung der Formel

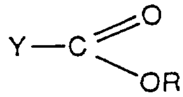

(I)

enthalten, in der

a. R für einen Rest steht, der von einem Duftalkohol der Formel ROH abgeleitet ist, und Y für einen linearen oder verzweigten, gesättigten oder ungesättigten $C_7$-$C_{24}$-Alkylrest oder für eine $-(CH_2)_nCOOR$-Gruppe steht, in der R wie obenstehend definiert ist und n eine ganze Zahl von 0 bis 6 ist;
oder
b. Y für einen linearen oder verzweigten, gesättigten oder ungesättigten $C_7$-$C_{24}$-Alkylrest steht und R für eine

Gruppe der Formel

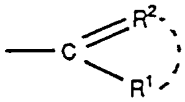

steht, in der entweder $R^1$ für Wasserstoff steht und $R^2$ für einen Alkylidenrest steht, der von einem Duftaldehyd der Formel

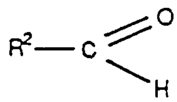

abgeleitet ist,
oder $R^2$ für einen Alkylidenrest und $R^1$ für einen Alkylrest steht, wobei $R^1$ und $R^2$ von einem Duftketon der Formel

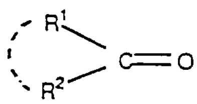

abgeleitet sind und Teil eines Rings sein können, wie durch die gestrichelte Linie angegeben ist, der 5 bis 18 Kohlenstoffatome enthält und gegebenenfalls substituiert ist.

**2.** Verfahren zum Beduften von Textilien, die einer Wäsche in Gegenwart eines eine Lipase enthaltenden Detergens unterzogen werden, wobei das Verfahren die Behandlung der Textilien nach dem Waschzyklus mit einem Textilweichspüler aufweist, der eine Verbindung der Formel (I) gemäß der Definition in Anspruch 1 enthält.

**3.** Verfahren zum Waschen von Textilien, das einen Waschzyklus in Gegenwart eines eine Lipase enthaltenden Detergens aufweist, gegebenenfalls gefolgt von einer Behandlung mit einem Textilweichspüler, wobei das Verfahren **dadurch gekennzeichnet ist, daß** das Detergens und/oder der Weichspüler eine Verbindung der Formel

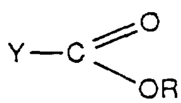

(I)

gemäß der Definition in Anspruch 1 enthalten.

**4.** Verfahren zum Waschen von Textilien, das einen Waschzyklus in Gegenwart eines eine Lipase enthaltenden Detergens aufweist, gefolgt von einer Behandlung der Textilien mit einem Textilweichspüler, der eine Verbindung der Formel (I) gemäß der Definition in Anspruch 1 enthält.

**5.** Verfahren zum Verlängern des Effekts des Verbreitens des charakteristischen Duftes eines Duftalkohols der Formel ROH, der von Textilien entwickelt wird, **dadurch gekennzeichnet, daß** die Textilien einem Waschzyklus in Gegenwart eines eine Lipase enthaltenden Detergens und gegebenenfalls einer nachfolgenden Behandlung mit einem Textilweichspüler unterzogen werden, wobei das Detergens und/oder der Weichspüler eine Verbindung der Formel

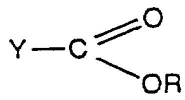

(I)

enthalten, in der R für einen von dem Duftalkohol abgeleiteten einwertigen Rest steht und Y für einen linearen oder verzweigten, gesättigten oder ungesättigten $C_7$-$C_{24}$-Alkylrest oder für eine -$(CH_2)_n$COOR-Gruppe steht, in der R wie obenstehend definiert ist und n eine ganze Zahl von 0 bis 6 ist.

**6.** Verfahren zum Verlängern des Effekts des Verbreitens des charakteristischen Duftes eines Duftalkohols der Formel ROH, der von Textilien entwickelt wird, die einer Wäsche mit einem eine Lipase enthaltenden Detergens unterzogen wurden, **dadurch gekennzeichnet, daß** die Textilien nach dem Waschzyklus mit einem Textilweichspüler behandelt werden, der eine Verbindung der Formel

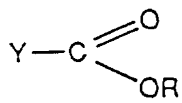

(I)

enthält, in der R für einen von dem Duftalkohol abgeleiteten einwertigen Rest steht und Y für einen linearen oder verzweigten, gesättigten oder ungesättigten $C_7$-$C_{24}$-Alkylrest oder für eine -$(CH_2)_n$COOR-Gruppe steht, in der R wie obenstehend definiert ist und n eine ganze Zahl von 0 bis 6 ist.

**7.** Verfahren zum Verlängern des Effekts des Verbreitens des charakteristischen Duftes eines Duftaldehyds der Formel

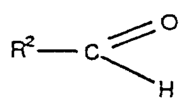

oder eines Duftketons der Formel

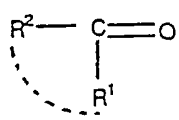

der von Textilien entwickelt wird, **dadurch gekennzeichnet, daß** die Textilien einem Waschzyklus in Gegenwart eines eine Lipase enthaltenden Detergens und gegebenenfalls einer nachfolgenden Behandlung mit einem Textilweichspüler unterzogen werden, wobei das Detergens und/oder der Weichspüler eine Verbindung der Formel

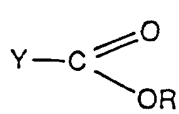

(I)

enthalten, in der Y für einen linearen oder verzweigten, gesättigten oder ungesättigten $C_7$-$C_{24}$-Alkylrest steht und R für eine Gruppe der Formel

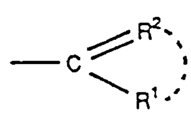

steht, in der entweder $R^1$ für Wasserstoff steht und $R^2$ für einen Alkylidenrest steht, der von dem Duftaldehyd abgeleitet ist, oder $R^2$ für einen Alkylidenrest und $R^1$ für einen Alkylrest steht, wobei $R^1$ und $R^2$ von dem Duftketon abgeleitet sind und Teil eines Rings sein können, wie durch die gestrichelte Linie angegeben ist, der 5 bis 18 Kohlenstoffatome enthält und gegebenenfalls substituiert ist.

**8.** Verfahren zum Verlängern des Effekts des Verbreitens des charakteristischen Duftes eines Duftaldehyds der Formel

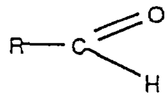

oder eines Duftketons der Formel

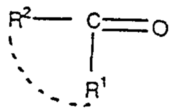

der von Textilien entwickelt wird, die einer Wäsche mit einem eine Lipase enthaltenden Detergens unterzogen wurden, **dadurch gekennzeichnet, daß** die Textilien nach dem Waschzyklus mit einem Textilweichspüler behandelt werden, der eine Verbindung der Formel

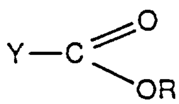

(I)

enthält, in der Y für einen linearen oder verzweigten, gesättigten oder ungesättigten $C_7$-$C_{24}$-Alkylrest steht und R für eine Gruppe der Formel

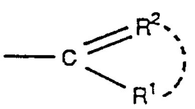

steht, in der entweder $R^1$ für Wasserstoff steht und $R^2$ für einen Alkylidenrest steht, der von dem Duftaldehyd abgeleitet ist, oder $R^2$ für einen Alkylidenrest und $R^1$ für einen Alkylrest steht, wobei $R^1$ und $R^2$ von dem Duftketon abgeleitet sind und Teil eines Rings sein können, wie durch die gestrichelte Linie angegeben ist, der 5 bis 18 Kohlenstoffatome enthält und gegebenenfalls substituiert ist.

**9.** Verfahren nach einem der Ansprüche 1 bis 6, welches die Zugabe einer Verbindung der Formel (I) aufweist, in der R der Rest Dec-9-en-1-yl, 3,7-Dimethyl-octa-2,6-dien-1-yl, 3,7-Dimethyl-oct-6-en-1-yl oder 3-Methyl-5-phenylpentyl ist.

**10.** Verfahren nach einem der Ansprüche 1 bis 4, 7 oder 8, welches die Zugabe einer Verbindung der Formel (I) aufweist, in der R der Rest 3,7-Dimethyl-1,6-octa-dienyl, 3-Methyl-5-phenyl-1-pentenyl oder 2-Methyl-1-undecenyl ist.

**11.** Verfahren nach einem der Ansprüche 1 bis 10, welches die Zugabe einer Verbindung der Formel (I) aufweist, in der Y ein linearer $C_{12}$-$C_{24}$-Alkylrest ist.

**12.** Detergens oder Textilweichspüler, das bzw. der eine Verbindung der Formel (I) gemäß der Definition von Anspruch 1 aufweist.

**13.** Verbindung der Formel

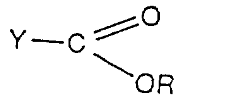

(I)

in der

a. R für einen Rest steht, der von einem Duftalkohol der Formel ROH abgeleitet ist, und Y für einen linearen oder verzweigten, gesättigten oder ungesättigten $C_7$-$C_{24}$-Alkylrest oder für eine -$(CH_2)_n$COOR-Gruppe steht, in der R wie obenstehend definiert ist und n eine ganze Zahl von 0 bis 6 ist;
oder
b. Y für einen linearen oder verzweigten, gesättigten oder ungesättigten $C_7$-$C_{24}$-Alkylrest steht und R für eine Gruppe der Formel

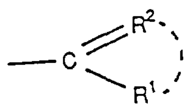

steht, in der entweder $R^1$ für Wasserstoff steht und $R^2$ für einen Alkylidenrest steht, der von einem Duftaldehyd der Formel

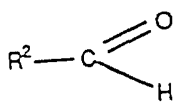

abgeleitet ist, oder $R^2$ für einen Alkylidenrest und $R^1$ für einen Alkylrest steht, wobei $R^1$ und $R^2$ von einem Duftketon der Formel

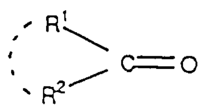

abgeleitet sind und Teil eines Rings sein können, wie durch die gestrichelte Linie angegeben ist, der 5 bis 18 Kohlenstoffatome enthält und gegebenenfalls substituiert ist, unter der Maßgabe, daß 2-Phenylethyloctanoat, n-Hexyladipat, Cyclohexyladipat und n-Hexylcyclohexyladipat ausgeschlossen sind.

**14.** Verbindung nach Anspruch 13, in der R der Rest Dec-9-en-1-yl, 3,7-Dimethyl-octa-2,6-dien-1-yl, 3,7-Dimethyl-oct-6-en-1-yl, 3-Methyl-5-phenylpentyl, 3,7-Dimethyl-1,6-octadienyl, 3-Methyl-5-phenyl-1-pentenyl oder 2-Methyl-1-undecenyl ist.

**15.** Verbindung nach Anspruch 13 oder 14, in der Y ein linearer $C_{12}$-$C_{24}$-Alkylrest ist.

**16.** Riechstoffzusammensetzung, welche mindestens einen Riechstoff, ein in der Parfümherstellung gängiges Lösungsmittel bzw. Zusatzmittel enthält, **dadurch gekennzeichnet, daß** sie eine Verbindung der Formel (I) gemäß der Definition in Anspruch 1 aufweist.

## Claims

1. A process for perfuming fabrics washed in the presence of a lipase-containing detergent and, optionally, subsequently treated with a fabric softener, said process being **characterized in that** said detergent and/or said fabric softener contains a compound of formula

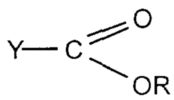

(I)

wherein

a. R represents a radical derived from a fragrant alcohol of formula ROH and Y represents a $C_7$ to $C_{24}$ linear or branched, saturated or unsaturated alkyl radical, or a $-(CH_2)_nCOOR$ group wherein R is defined as above and n is an integer from 0 to 6 ; or
b. Y represents a $C_7$ to $C_{24}$ linear or branched, saturated or unsaturated alkyl radical and R represents a group of formula

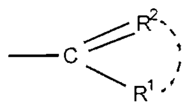

wherein, either $R^1$ represents hydrogen and $R^2$ represents an alkylidene radical derived from a fragrant aldehyde of formula

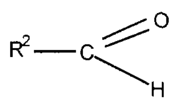

or $R^2$ represents an alkylidene radical and $R^1$ an alkyl radical, $R^1$ and $R^2$ being then derived from an fragrant ketone of formula

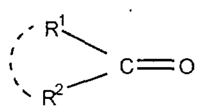

and, optionally, being part of a ring such as indicated by the dotted line which contains 5 to 18 carbon atoms and can be substituted.

2. A process for perfuming fabrics washed in the presence of a lipase-containing detergent, which process comprises the treatment of said fabrics, after the washing cycle, with a fabric softener containing a compound of formula (I) as defined in claim 1.

3. A process for washing fabrics, comprising a washing cycle in the presence of a lipase-containing detergent, optionally followed by a treatment with a fabric softener, said process being **characterized in that** said detergent and/or said fabric softener contains a compound of formula

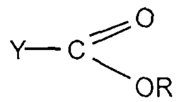

(I)

as defined in claim 1.

**4.** A process for washing fabrics, comprising a washing cycle in the presence of a lipase-containing detergent, followed by the treatment of said fabrics with a fabric softener containing a compound of formula (I) as defined in claim 1.

**5.** A process for prolonging the diffusion effect of the odour developed by fabrics and which is characteristic of a fragrant alcohol of formula ROH, said process being **characterized in that** the fabrics undergo a washing cycle in the presence of a lipase-containing detergent and, optionally, a subsequent treatment with a fabric softener, said detergent and/or said fabric softener containing a compound of formula

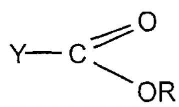

(I)

wherein R represents a monovalent radical derived from said fragrant alcohol, and Y represents a $C_7$ to $C_{24}$ linear or branched, saturated or unsaturated alkyl radical, or a $-(CH_2)_nCOOR$ group wherein R is defined as above and n is an integer from 0 to 6.

**6.** A process for prolonging the diffusion effect of the odour characteristic of a fragrant alcohol of formula ROH and which is developed by fabrics washed with a lipase-containing detergent, said process being **characterized in that** said fabrics, after the washing cycle, are treated with a fabric softener containing a compound of formula

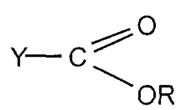

(I)

wherein R represents a monovalent radical derived from said fragrant alcohol, and Y represents a $C_7$ to $C_{24}$ linear or branched, saturated or unsaturated alkyl radical, or a $-(CH_2)_nCOOR$ group wherein R is defined as above and n is an integer from 0 to 6.

**7.** A process for prolonging the diffusion effect of the odour developed by fabrics and which is characteristic of a fragrant aldehyde of formula

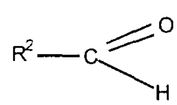

or of a fragrant ketone of formula

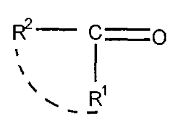

said process being **characterized in that** the fabrics undergo a washing cycle in the presence of a lipase-containing detergent and, optionally, a subsequent treatment with a fabric softener, said detergent and/or said fabric softener

containing a compound of formula

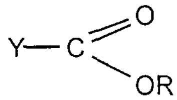 (I)

wherein Y represents a $C_7$ to $C_{24}$, linear or branched, saturated or unsaturated alkyl radical, and R represents a group of formula

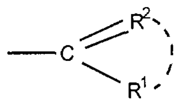

wherein, either $R^1$ represents hydrogen and $R^2$ represents an alkylidene radical derived from said fragrant aldehyde, or $R^2$ represents an alkylidene radical and $R^1$ an alkyl radical, $R^1$ and $R^2$ being then derived from said fragrant ketone and, optionally, being part of a ring such as indicated by the dotted line which contains from 5 to 18 carbon atoms and can be substituted.

**8.** A process for prolonging the diffusion effect of the odour characteristic of a fragrant aldehyde of formula

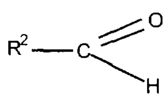

or of a fragrant ketone of formula

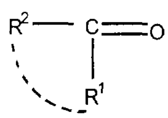

developed by fabrics subjected to washing with a lipase-containing detergent, said process being **characterized in that** said fabrics, after the washing cycle, are treated with a fabric softener containing a compound of formula

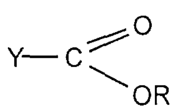 (I)

wherein Y represents a $C_7$ to $C_{24}$, linear or branched, saturated or unsaturated alkyl radical, and R represents a group of formula

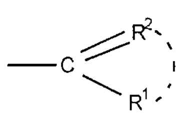

wherein, either $R^1$ represents hydrogen and $R^2$ represents an alkylidene radical derived from said fragrant aldehyde, or $R^2$ represents an alkylidene radical and $R^1$ an alkyl radical, $R^1$ and $R^2$ being then derived from said fragrant ketone and, optionally, being part of a ring such as indicated by the dotted line which contains from 5 to 18 carbon atoms and can be substituted.

**9.** A process according to one of claims 1 to 6, comprising the addition of a compound of formula (I) wherein R is the 9-decen-1-yl, 3,7-dimethyl-2,6-octadien-1-yl, 3,7-dimethyl-6-octen-1-yl or 3-methyl-5-phenylpentyl radical.

**10.** A process according to one of claims 1 to 4, 7 or 8, comprising the addition of a compound of formula (I) wherein R is the 3,7-dimethyl-1,6-octadienyl, 3-methyl-5-phenyl-1-pentenyl or 2-methyl-1-undecenyl radical.

**11.** A process according to one of claims 1 to 10, comprising the addition of a compound of formula (I) wherein Y is a $C_{12}$ à $C_{24}$ linear alkyl radical.

**12.** A detergent or a fabric softener comprising a compound of formula (I) as defined in claim 1.

**13.** A compound of formula

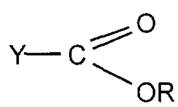

(I)

wherein

a. R represents a radical derived from an fragrant alcohol of formula ROH and Y represents a $C_7$ to $C_{24}$ linear or branched, saturated or unsaturated alkyl radical, or a $-(CH_2)_nCOOR$ group wherein R is defined as above and n is an integer from 0 to 6 ; or

b. Y represents a $C_7$ to $C_{24}$ linear or branched, saturated or unsaturated, alkyl radical and R represents a group of formula

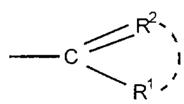

wherein, either $R^1$ represents hydrogen and $R^2$ represents an alkylidene radical derived from a fragrant aldehyde of formula

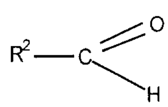

or $R^2$ represents an alkylidene radical and $R^1$ an alkyl radical, $R^1$ and $R^2$ being then derived from an fragrant ketone of formula

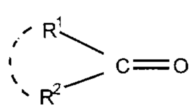

and, optionally, being part of a ring such as indicated by the dotted line which contains 5 to 18 carbon atoms and can be substituted.

**14.** A compound according to claim 13, wherein R is the 9-decen-1-yl, 3,7-dimethyl-2,6-octadien-1-yl, 3,7-dimethyl-6-octen-1-yl, 3-methyl-5-phenylpentyl, 3,7-dimethyl-1,6-octadienyl, 3-methyl-5-phenyl-1-pentenyl or 2-methyl-1-undecenyl radical.

**15.** A compound according to claim 13 or 14, wherein Y is a $C_{12}$ to $C_{24}$ linear alkyl radical.

**16.** A perfuming composition containing at least a perfuming ingredient, a solvent or adjuvant of common use in perfumery, **characterized in that** it contains a compound of formula (I) as defined in claim 1.